# NEW EUROPEAN PATENT SPECIFICATION

(11) **EP 0 619 111 B2**
(45) Date of publication and mention of the opposition decision: **14.09.2005**
(45) Mention of the grant of the patent: 27.12.1996
(21) Application number: 94105264.9
(22) Date of filing: 05.04.1994
(51) Int. Cl.: A61K 7/11

(54) **Use of polyurethanes with carboxylate functionality for hair fixative applications**
Verwendung von Polyurethanen, die funktionelle Carboxylatogruppe enthalt, zur Haarfestigung
Utilisation des polyuréthanes avec des groupes carboxyliques fonctionelle comme agent fixateur des cheveux

(30) Priority: 06.04.1993 US 43241
(43) Date of publication of application: 12.10.1994
(73) Proprietor: National Starch and Chemical Investment Holding Corporation, Wilmington, Delaware 19809 (US)
(72) Inventor: Thomaides, John, Berkely Heights, New Jersey 07922 (US); Russo, Julie V., Piscataway, New Jersey 08854 (US); Martino, Gary T., Plainsboro, New Jersey 08536 (US); Ray-Chaudhuri, Dilip, Bridgewater, New Jersey 08807 (US)
(74) Representative: Hagemann, Heinrich

(56) References cited:
- WO-A-94/03510
- US-A- 4 743 673
- Rompps Chemie-Lexikon,8 Auflage, Seite 961(1981)

## Description

### Description pour les Etats contractants suivants: BE, DE, FR, GB, IT

This invention pertains to hair fixative compositions that are prepared from polyurethanes containing pendant free carboxyl groups that are neutralized with standard cosmetically acceptable bases.

Most hair fixative compositions contain a film-forming polymer, which acts as the fixative, and a delivery system, which is usually an alcohol or a mixture of alcohol and water. In the case of aerosol delivery, the delivery system will also contain a propellant, which is typically a volatile hydrocarbon. Due to environmental regulations controlling the emission of volatile organic compounds (VOCs) into the atmosphere, these alcohol and hydrocarbon delivery systems are becoming less acceptable, and it is foreseen that water will become a greater component in hair fixative compositions. However, many hair fixative polymers in current use exhibit a loss of performance properties in aqueous based systems; for example, the solution viscosity increases, and if delivered by aerosol, the composition foams at the valve actuator and on the hair. These factors have prompted the search for hair fixative polymers that are soluble in aqueous or in low VOC systems, which are systems that contain 80% or less VOCs, and that perform with all the desired characteristics of a good hair fixative polymer, namely, good holding power, high humidity curt retention, quick drying time, nonstickiness, and a clear, transparent, glossy film that is easily removable with water or with water and shampoo.

This invention is an aqueous based hair fixative composition that comprises an effective amount of a polyurethane to perform as a hair fixative in an all water or in an alcohol-water solvent system. Specifically, the hair fixative composition comprises (A) an effective amount of a fully reacted carboxylated linear polyurethane comprising the reaction product of (i) one or more 2,2-hydroxymethyl-substituted carboxylic acids present in an amount to give 0.35-2.25 milliequivafents of carboxyl functionality per gram of polyurethane, (ii) 10-90% by weight, based on the weight of the polyurethane, of one or more organic compounds having no more than two active hydrogen atoms each, and (iii) one or more organic diisocyanates selected from the group consisting of methylenedi-p-phenyl diisocyanate and methylene-bis-(4 cyclohexylisocyanate) present in a sufficient amount to react with the active hydrogens of the 2,2-hydroxymethyl-substituted carboxylic acids and the organic compounds, excepting the hydrogen on the carboxylate of the 2,2-hydroxymethyl-substituted carboxylic acid; (8) an effective amount of a cosmetically acceptable organic or inorganic base to neutralize a sufficient proportion of the available carboxyl groups on the polyurethane to make the polyurethane soluble in water or in a mixture of water and a polar organic solvent; and (C) a solvent comprising (i) water, and (ii) 0-85% by weight of a polar organic solvent, based on the weight of the solvent. In aerosol systems, the hair fixative composition will further comprise up to 60% by weight of a propellant based on the weight of the total hair fixative composition.

The polyurethanes suitable for use in hair fixative formulations according to this invention are fully reacted carboxylated linear polymers. These polyurethanes are used in an effective amount to achieve hair holding and humidity resistance properties. They are preferably present in amounts from 1-20% by weight of the hair fixative composition, and more preferably in amounts from 1-10% by weight. These polyurethanes are the reaction products of (i) one or more 2,2-hydroxymethyl-substituted carboxylic acids present in an amount to give 0.35-2.25 milliequivalents, preferably 0.5-1.85 milliequivalents, of carboxyl fundionality per gram of polyurethane, (ii) 10-90% by weight, based on the weight of the polyurethane, of one or more organic compounds having no more than two active hydrogen atoms each, and (iii) one or more organic diisocyanates selected from the group consisting of methylenedi-p-phenyl diisocyanate and methylene-bis-(4 cyclohexylisocyanate) present in a sufficient amount to react with the active hydrogens of the 2,2-hydroxymethyl-substituted carboxylic acid and the organic compounds, excepting the hydrogen on the carboxylate of the 2,2-hydroxymethyl-substituted carboxylic acid. The incorporation of the 2,2-hydroxymethyl-substituted carboxylic acid introduces pendant carboxylic acid groups into the polymer chain, which after neutralization render the polyurethane soluble in water and in mixtures of water with other polar solvents. Using these polyurethanes as the active ingredient, hair fixative formulations can be made that have a high solids content with low viscosity. A high solids content supplies an effective amount of polymer to the hair in a minimum amount of solvent to obtain good holding power. Low viscosity permits effective atomization at the spray nozzle. Thus, a hair fixative product suitable for use in either aerosol or nonaerosol formulations can be achieved. The use of 2,2-hydroxymethyl-substituted carboxylic acids also imparts increased film hardness and rigidity to the polyurethane, properties that are desirable for hair fixatives.

The 2,2-hydroxymethyl-substituted carboxylic acids are represented by the formula 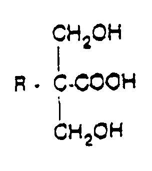 in which R represents hydrogen, or C₁ - C₂₀ alkyl, preferably C₁ - C₈ alkyl. Specific examples include 2,2-di(hydroxymethyl)acetic acid, 2,2-di(hydroxymethyl)propionic acid, 2,2-di-(hydroxymethyl)butyric acid, 2,2-di(hydroxymethyl)pentanoic acid, and the like. The preferred acid is 2,2-di-(hydroxymethyl)propionic acid. The 2.2-hydroxymethyl-substituted carboxylic acids are present in an amount to give 0.35-2.25, preferably 0.5-1.85, milliequivalents of carboxyl functionality per gram of polyurethane, and in general this is about 5-30% by weight of the polyurethane polymer.

The organic compounds that are reactive with isocyanate and that may be used for the preparation of the polyurethane polymers of this invention have no more than two active hydrogen atoms (as determined by the Zerewitinoff method). The active hydrogen atoms are usually attached to oxygen, nitrogen or sulfur atoms. These compounds will have a molecular weight of about 300 to 20,000, preferably about 500 to 8,000. Preferably, these compounds will be linear in order to prevent gelling during polymerization, but small amounts of non-linear compounds may be used provided their use does not cause gelling. The organic compounds will be present in an amount of 10-90% by weight of the polyurethane, preferably in an amount of 15-70% by weight.

The preferred organic compounds with two active hydrogen atoms are the linear difunctional polyethylene and polypropylene glycols, especially those that are available commercially and produced by the reaction of ethylene (or propylene) oxide with water, ethylene (or propylene) glycol, or diethylene (or dipropylene) glycol in the presence of sodium hydroxide as a catalyst. These polyglycols have molecular weights of about 600 to 20,000, preferably about 1,000 to 8,000. Polyglycols that are homogeneous in molecular weight, or a mixture of glycols that differ in molecular weight can be used. It is also possible to copolymerize small amounts of additional alkylene oxides into the polyglycol.

Other suitable organic compounds with two active hydrogen atoms are those having hydroxyl, carboxyl, amino or mercapto groups. Among these, the preferred are polyhydroxy compounds, such as, polyether diols, polyester diols, polyacetal diols, polyamide diols, polyester polyamide diols, poly(alkylene ether)diols, polythioether diols, and polycarbonate diols. Compounds that contain two or more different groups within these classes may also be used, for example, amino alcohols and amino alcohols containing two amino groups and one hydroxyl group. It is preferred to use difunctional compounds although small amounts of tri-(and greater) functional compounds may be used.

Suitable polyether diols are, for example, the condensation products of ethylene oxide, propylene oxide, butylene oxide, or tetrahydrofuran, and their copolymerization, graft or block polymerization products, such as, mixed ethylene oxide, propylene oxide condensates, and the graft polymerization products of the reaction of olefins under high pressure with the mentioned alkylene oxide condensates. Suitable polyethers are prepared by the condensation of the mentioned alkylene oxides with polyhydric alcohols, such as, ethylene glycol, 1,2-propylene glycol and 1,4-butanediol.

Suitable polyester diols, polyester amide diols, and polyamide diols are preferably saturated, and are obtained, for example, from the reaction of saturated or unsaturated polycarboxylic acids with saturated or unsaturated polyhydric alcohols, diamines, or polyamines. Suitable carboxylic acids for preparing these compounds include, for example, adipic acid, succinic acid, phthalic acid, terephthalic acid, and maleic acid. Suitable polyhydric alcohols for preparing the polyesters include, for example, ethylene glycol, 1,2-propylene glycol, 1,4-butanediol, neopentyl glycol, and hexanediol. Amino alcohols, for example, ethanolamine, are also useful. Suitable diamines for preparing the polyester amides and polyamides are, for example, ethylene diamine and hexamethylene diamine.

Suitable polyacetals can be prepared, for example, from 1,4-butanediol or hexanediol and formaldehyde. Suitable polythioethers can be prepared, for example, as the condensation products of thiodiglycol either alone or in combination with other glycols, such as, ethylene glycol, 1,2-propylene glycol or with other polyhydroxy compounds as disclosed above. Polyhydroxy compounds that already contain urea or urethane groups, and natural polyols, which may be further modified, for example, castor oil and carbohydrates, may also be used.

In preparing the polyurethane polymer, in addition to the organic compound having no more than two active hydrogen atoms, which in many cases is a high molecular weight compound, it may be desirable to chain extend the polymer using an organic compound with a lower molecular weight, preferably less than about 300 and more than 60. Typical chain extending agents include saturated or unsaturated glycols, such as, ethylene glycol, diethylene glycol, triethylene glycol and the like; amino alcohols, such as, ethanolamine, propanolamine, butanolamine, and the like; mono- and dialkoxylated aliphatic, cycloaliphatic, aromatic and heterocyclic primary amines, such as. N-methyidiethanoiamine, N-oleyl diethanolamine, N-cyclohexyl diisopropanelamine. N,N-dihydroxyethyl-p-toluidine, N,N-dihydroxy-propylnaphthylamine and the like; diamines, such as ethylene diamine, piperazine, N-N-bis-gamma-aminopropyl-N-methyl-amine and the like; carboxylic acids including aliphatic, cycloaliphatic, aromatic and heterocyclic dicarboxylic acids, such as, oxalic acid, malonic acid, succinic acid, glutaric acid, adipic acid, sebacic acid, terephthalic acid, 1,5-dicarboxylic naphthalic acid, maleic acid, fumaric acid, diglycolic acid, quinolinic acid, lutidinic acid and the like; amino carboxylic acids, such as, glycine, alpha and beta-alanine, 6-aminocaproic acid, 4-aminobutyric acid, p-aminobenzoic acid, 5-aminonaphthoic acid and the like. The preferred chain extending agents are aliphatic diols.

The organic polyisocyanates or mixtures of polyisocyanates that are reacted with the organic compound are selected from the group consisting of methylenedi-p-phenyl diisocyanate and methylene-bis-(4-cyclohexylisocyanate). The isocyanate will be present in a sufficient amount to react with the active hydrogens of the 2,2-hydroxymethyl-substituted carboxylic acid and the organic compounds, excepting the hydrogen on the carboxylate of the 2,2-hydroxymethyl-substituted carboxylic acid. This amount will vary depending on the amounts of the carboxylic acid and organic compounds.

If is desired not to chain extend the polymer, the reaction of the diisocyanate with the organic compound having two active hydrogen atoms is quenched by the addition of a monofunctional active hydrogen-containing compound to consume any residual isocyanate functionality. Examples of these quenching compounds are well known in the art; for these systems, the preferred quenching compound is ethanol.

The urethane polymerization is carried out in the reaction medium with or without typical urethane reaction catalysts known in the art. Suitable catalysts include dibutyl tin dilaurate; the stannous salts of carboxylic acids having from 2 to 18 carbon atoms, such as, stannous laurate, stannous stearate, stannous acetate, stannous butyrate, stannous octoate and the like, and mixtures of those. Other suitable catalysts include dibutyl tin oxide, dibutyl tin sulfide, lead resinate, lead benzoate, lead salicytate, lead 2-ethyl hexoate, lead oleate, iron acetyl acetonate, cobalt benzoate, tetra (2-ethyl hexyl) titanate, tetra butyl titanate, and the like. Many other compounds accelerate the reaction of a hydroxyl or other groups with an isocyanate in preference to certain other reactions of the isocyanate group, and any of these compounds may be used. Those skilled in the art will choose a specific catalyst to confer desired characteristics to individual urethane reactions. The preceding specific compounds are the preferred compounds and are given for the purpose of illustration and not limitation. In addition, any suitable tertiary amine may be used alone or with the metallic catalyst, for example, triethylene diamine, N-ethyl morpholine, N-methyl morpholine, or 4-dimethyl amino ethyl piperazine.

With respect to the proportion of reactants, the reactants should be selected so that the molecular ratio of isocyanate groups to active hydrogen atoms is as close to 1:1 as is practicable. It is appreciated that this exact ratio may not always be attained in practice; therefore, a ratio between about 0.7:1 and 1.3:1, and preferably between about 0.9:1 and 1.2:1, should be sought, and any excess diisocyanate, as discussed previously, can be quenched with the mono-functional active hydrogen containing compound.

The polymerization is carried out according to well known polyurethane polymerization techniques, which are well known to those skilled in the art. Exemplary polymerizations and reaction conditions are given in the examples.

To be used as hair fixatives, the carboxylated polyurethanes must be capable of being removed from the hair after use by a water rinse or shampoo. Removability is imparted by neutralizing the free carboxyl groups on the polyurethane. The amount of base used for neutralization is dependent on the hydrophobicity of the hair fixative polymer. The higher the carboxylic acid content of the polymer, the less the degree of neutralization required to impart water solubility. Conversely, the lower the carboxylic acid content, the greater the degree of neutralization required for water solubility. The preferred levels of neutralization range from 50-90%, depending on the acidity of the polymer. Suitable bases for neutralization of the polymer are the standard cosmetically acceptable bases known and used in the art. The preferred bases are sodium hydroxide, potassium hydroxide. 2-amino-2-methyl-1-propanol, histidine, tris(hydroxymethyl)aminomethane, and triethanoiamine. The choice of the base and the degree of neutralization also affect the flexibility of the resultant hair fixative when sprayed on the hair, giving a soft or a hard hold. The choice of which base to utilize and the degree of neutralization required to achieve flexibility is within the expertise of one skilled in the art. In general, however, the amount of base for neutralization will be within the range of 0.05-0.80% based on the total weight of the compcsition, although it will be recognized that individual formulations may require neutralization outside this range.

The neutralized polymers are soluble in water and, thus, the hair fixative compositions may be based solely in water, although more typically, the solvent system will be a blend of polar organic solvent and water. Typically, the organic solvent will be an alcohol or ketone. Particularly suitable solvents are low boiling alcohols that are compatible with other components in the hair fixative composition, for example, C₁-C₄ straight or branched chain alcohols. Exemplary polar solvents are ethanol, propanol, isopropanol, butanol, and acetone.

Hair fixative compositions that are intended to be delivered in an aerosol systems additionally will require a propellant. While any of the known propellants may be used in these compositions, preferred propellants include the hydrocarbons, particularly the lower boiling hydrocarbons such as C₃-C₆ straight and branched chain hydrocarbons, for example, propane, butane, isobutane and mixtures of those. Other preferred propellants include the ethers, such as dimethyl ether; hydrofluorocarbons, such as, 1,1-difluoroethane; and the compressed gases, such as nitrogen, air and carbon dioxide. The amount of propellant used in the hair fixative compositions of this invention may vary from about 0 to 60% by weight of the hair spray composition and preferably from about 0 to 40% by weight, based on the weight of the total composition.

An important consideration in determining the amount of organic solvent, or organic solvent and propellant, to be used in the hair fixative composition is the total amount of volatile organic compound (VOC) content, and any upper limit of VOC content that may be mandated by environmental regulations. While these compositions may have a wide range of VOC content, from 0 to 85% by weight, it is preferred that there be less than about 80% and more preferably less than about 55% by weight VOC content, based on the weight of the composition. The balance of the hair fixative composition will be water and the neutralized polyurethane.

Optional conventional additives may also be incorporated into the hair fixing composition of this invention to provide certain modifying properties to the composition. Included among these additives are plasticizers, such as glycerine, glycol and phthalate esters: silicones; emollients, lubricants and penetrants, such as lanolin compounds; fragrances and perfumes; UV absorbers; dyes and other colorants; thickeners; anticorrosion agents; detackifying agents; combing aids; antistatic agents; preservatives; and foam stabilizers. These additives are present in small, effective amounts to accomplish their function, and generally will comprise from about 0.1 to 10% by weight each, and from about 0.1 to 20% by weight total, based on the weight of the composition.

The resulting hair fixing compositions exhibit all of the characteristics required of such a product in systems ranging from 0 to 85% VOC. The films found are clear, hard, glossy and provide humidity resistance while being readily removable.

### Examples

The following examples disclose the preparation of polyurethanes containing varying levels of dimethytol propionic acid and the results of humidity resistance testing on hair fixative formulations prepared from those polyurethanes.

### Syntheses of Polyurethanes

Polyurethane A. A polyurethane containing 16.7% by weight dimethylol propionic acid was prepared (total carboxylate functionality: 1.25 meq/g). The product was isolated as an emulsion in water.

| Reagents: | | |
|---|---|---|
| 1a. | Polypropylene glycol (1000 molecular weight) | 34.12 g |
| 1b. | Polypropylene glycol (3000 molecular weight) | 47.36 g |
| 2. | Polyethylene glycol (8000 molecular weight) | 10.00 g |
| 3. | Acetone (anhydrous) | 200.0 g |
| 4. | Methytenedi-p-phenyl diisocyanate (260 effective MW) | 75.12 g |
| 5. | Dibutyl tin dilaurate | 0.20 g |
| 6. | Dimethylol propionic acid | 33.40 g |
| 7. | Ethanol | 5.0 g |
| 8. | 1.0 N Sodium Hydroxide | 100.0 mL |
| 9. | Deionized water as needed to bring reagent 8 to 300g | |

A 1 L four-neck round bottom flask equipped with three stoppers and a vacuum adapter was charged with reagents 1 and 2. The reaction mixture was heated to 110°C under dynamic vacuum (<1 mm Hg) for 1 hour. The vacuum was released with nitrogen, and the flask was fit with a mechanical stirrer, thermometer, stopper, and a gas inlet-topped condenser. The reaction vessel was placed under a positive pressure of nitrogen and was maintained in this manner throughout the course of the urethane condensation reaction. The vessel was charged with reagent 3. The resulting mixture was brought to reflux and held there until a homogenous solution was obtained.

The reaction mixture was allowed to cool to less than 50°C. Reagents 4 and 5 were added, and the reaction was brought to reflux. The reaction was allowed to stir at reflux for 5 hours. Reagent 6 was added, and the reaction was stirred at reflux for an additional 11 hours. Reagent 7 was then added to the reaction mixture, and the reaction was allowed to stir at reflux for 1 hour.

Reagent 8 was charged into an addition funnel and then a sufficient quantity of reagent 9 was added to bring the total weight of reagent in the addition funnel to 300g. The contents of the funnel were added to the reaction mixture uniformly over a 3 hour period at 60°C. When the addition was complete, the flask was equipped for simple distillation, and distillation of solvent from the reaction mixture was started. When the pot temperature reached 90°C, steam distillation of the reaction mixture via the introduction of sub-surface steam was commenced. The steam distillation was continued until the pot temperature reached 100°C and was maintained at 100°C for 1 hour. A stable, fully aqueous emulsion was obtained at the conclusion of the steam distillation. The yield was 524.3g (37.6% solids). The neutralization equivalent of the emulsion was 0.286 meq/g. This corresponds to a neutralization equivalent of 0.761 meq/g on a 100% solids basis.
Polyurethane B. A polyurethane containing 14.6% by weight dimethylol propionic acid (total carboxylate functionality: 1.09 meq/g) and 2% by weight N-methyl diethanolamine was prepared. This polyurethane contains both cationic and anionic functionality. The product was isolated as an emulsion in water.

| Reagents: | | |
|---|---|---|
| 1. | Polyethylene glycol (2000 molecular weight) | 51.30 g |
| 2. | Polyethylene glycol (8000 molecular weight) | 16.57 g |
| 3. | Dimethylol propionic acid | 21.95 g |
| 4. | 2-Butanone | 190.4 g |
| 5. | Methylenedi-p-phenyl diisocyanate (260 effective MW) | 57.23 g |
| 6. | Dibutyl tin dilaurate | 0.20 g |
| 7. | N-methyl diethanolamine | 3.09 g |
| 8. | Ethanol | 5.0 g |
| 9. | 1.0 N Sodium hydroxide | 75.0 mL |
| 10. | Deionized water as needed to bring reagent 9 to 325g | |

A 1 L four-neck round bottom flask was equipped with a mechanical stirrer, thermometer, stopper, and a Dean & Stark-type receiver with draw-off valve fit with a gas inlet-topped condenser. The reaction vessel was placed under a positive pressure of nitrogen and was maintained in this manner throughout the course of the urethane condensation reaction. The vessel was charged with reagents 1, 2, 3, and 4. The resulting suspension was brought to reflux, and a total of 39.9g 2-butanone was distilled from the reaction vessel via the Dean & Stark receiver.

The reaction mixture was allowed to cool to less than 40°C. Reagents 5 and 6 added, and the reaction temperature was brought to 60°C. The reaction was allowed to stir at 60°C for 19 hours at which time the residual isocyanate was found to be 0.77% (theory: 0.81%). Reagent 7 was added at this time, and the reaction was stirred at 60°C for an additional 1.5 hours. The residual isocyanate was re-measured and found to be 0.11% (theory: 0.07%). Reagent 8 was then added to the reaction mixture, and the reaction was allowed to stir at 60°C for 1 hour.

Reagent 9 was charged into an addition funnel and then a sufficient quantity of reagent 10 was added to bring the total weight of reagent in the addition funnel to 325g. The contents of the funnel were added to the reaction mixture uniformly over a 3 hour period at 60°C. When the addition was complete, simple distillation at atmospheric pressure of solvent from the reaction mixture was started. When the pot temperature reached 90°C, steam distillation of the reaction mixture via the introduction of sub-surface steam was commenced. The steam distillation was continued until the pot temperature reached 100°C and was maintained at 100°C for 1 hour. A stable, fully aqueous emulsion was obtained at the conclusion of the steam distillation. The yield was 495.8g (28.6% solids). The neutralization equivalent of the emulsion was 0.167 meq/g. This corresponds to a neutralization equivalent of 0.584 meq/g on a 100% solids basis.
Polyurethane C. A polyurethane containing 24.6% by weight dimethylol propionic acid was prepared (total carboxylate functionality: 1.83 meq/g). The product was isolated as an emulsion in water.

| Reagents: | | |
|---|---|---|
| 1. | Polypropylene glycol (2000 molecular weight) | 21.47 g |
| 2. | Polyethylene glycol (8000 molecular weight) | 16.50 g |
| 3. | Dimethytol propionic acid | 36.31 g |
| 4. | 2-Butanone | 336.2 g |
| 5. | Methylenedi-p-phenyl diisocyanate (260 effective MW) | 75.83 g |
| 6. | Dibutyl tin dilaurate | 0.2 g |
| 7. | Ethanol | 5.0 g |
| 8. | 1.0 N Sodium Hydroxide | 100.0 mL |
| 9. | Deionized water as needed to bring reagent 8 to 300g | |

A 1 L four-neck round bottom flask was equipped with a mechanical stirrer, thermometer, stopper, and a Dean & Stark-type receiver with draw-off valve fit with a gas inlet-topped condenser. The reaction vessel was placed under a positive pressure of nitrogen and was maintained in this manner throughout the course of the urethane condensation reaction. The vessel was charged with reagents 1, 2, 3, and 4. The resulting suspension was brought to reflux, and a total of 43g 2-butanone was distilled from the reaction vessel via the Dean & Stark receiver.

The reaction mixture was allowed to cool to less than 40°C. Reagents 5 and 6 were added, and the reaction temperature was brought to 60°C. The reaction was allowed to stir at 60°C for 20 hours at which time the residual isocyanate was found to be 0.05% (theory: 0.15%). Reagent 7 was added at this time, and the reaction was stirred at 60°C for an additional hour.

Reagent 8 was charged into an addition funnel and then a sufficient quantity of reagent 9 was added to bring the total weight of reagent in the addition funnel to 300g. The contents of the funnel were added to the reaction mixture uniformly over a 3 hour period at 60°C. When the addition was completed, simple distillation at atmospheric pressure of solvent from the reaction mixture was started. When the pot temperature reached 90°C, steam distillation of the reaction mixture via the introduction of sub-surface steam was commenced. The steam distillation was continued until the pot temperature reached 100°C and was maintained at 100°C for 45 minutes. A stable, fully aqueous emulsion was obtained at the conclusion of the steam distillation. The yield was 401.3g (34.1% solids). The neutralization equivalent of the emulsion was 0.286 meq/g. This corresponds to a neutralization equivalent of 0.839 meq/g on a 100% solids basis.
Polyurethane D. A polyurethane containing 7.5% by weight dimethylol propionic acid was prepared (total carboxylate functionality: 0.56 meq/g). The product was isolated as an emulsion in water.

| Reagents: | | |
|---|---|---|
| 1. | Polypropylene glycol (2000 molecular weight) | 87.55 g |
| 2. | Polyethylene glycol (8000 molecular weight) | 16.48 g |
| 3. | Dimethylol propionic acid | 11.26 g |
| 4. | 2-Butanone | 190.7 g |
| 5. | Methylenedi-p-phenyl diisocyanate (260 effective MW) | 34.68 g |
| 6. | Dibutyl tin dilaurate | 0.2 g |
| 7. | Ethanol | 5.0 g |
| 8. | 0.94 N Sodium Hydroxide | 35.8 mL |
| 9. | Deionized water as needed to bring reagent 8 to 275g | |

The emulsion was prepared following the procedure outlined for polyurethane Q. The yield was 611g (23.9% solids). The neutralization equivalent of the emulsion was 0.080 meq/g. This corresponds to a neutralization equivalent of 0.33 meq/g on a 100% solids basis.
Polyurethane E. A polyurethane containing 14.6% by weight dimethyl propionic acid was prepared (total carboxylate functionality: 1.09 meq/g). A cycloaliphatic diisocyanate was used in the synthesis. The product was isolated as an emulsion in water.

| Reagents: | | |
|---|---|---|
| 1. | Polypropylene glycol (2000 molecular weight) | 63.48 g |
| 2. | Polyethylene glycol (8000 molecular weight) | 11.27 g |
| 3. | Dimethylol propionic acid | 22.03 g |
| 4. | 2-Butanone | 183.0 g |
| 5. | Methylene bis-(4-cyclohexyl isocyanate) (264 effective MW) | 53.90 g |
| 6. | Dibutyl tin dilaurate | 0.5 g |
| 7. | Ethanol | 10 g |
| 8. | 0.94 N Sodium Hydroxide | 34.6 mL |
| 9. | Deionized water as needed to bring reagent 8 to 275g | |

A 1 L four-neck round bottom flask was equipped with a mechanical stirrer, thermometer, stopper, and a Dean & Stark-type receiver with draw-off valve fit with a gas-inlet-topped condenser. The reaction vessel was placed under a positive pressure of nitrogen and was maintained in this manner throughout the course of the urethane condensation reaction. The vessel was charged with reagents 1, 2, 3, and 4. The resulting suspension was brought to reflux, and a total of 37.5g 2-butanone was distilled from the reaction vessel via the Dean & Stark receiver.

The reaction mixture was allowed to cool to less than 60°C. Reagents 5 and 6 were added, and the reaction temperature was brought to 70°-75°C. The reaction was allowed to stir at 70°-75°C for 30 hours at which time the residual isocyanate was found to be 0. 26% (theory: 0.20%). Reagent 7 was added at this time, and the reaction was stirred at 70°-75°C for an additional 10 hours.

Reagent 8 was charged into an addition funnel and then a sufficient quantity of reagent 9 was added to bring the total weight of reagent in the addition funnel to 275g. The contents of the funnel were added to the reaction mixture uniformly over a 3 hour period at 60°C. When the addition was complete, simple distillation at atmospheric pressure of solvent from the reaction mixture was started. When the pot temperature reached 90°C, steam distillation of the reaction mixture via the introduction of sub-surface steam was commenced. The steam distillation was continued until the pot temperature reached 100°C and was maintained at 100°C for 30 minutes. A stable, fully aqueous emulsion was obtained at the conclusion of the steam distillation. The yield was 458.1g (31.1% solids). The neutralization equivalent of the emulsion was 0.257 meq/g. This corresponds to a neutralization equivalent of 0.826 meq/g on a 100% solids basis.
Polyurethane F. A polyurethane containing 16.7% by weight dimethylol propionic acid was prepared (total carboxylate functionality: 1.25 meq/g). The product was isolated as an emulsion in water.

| Reagents: | | |
|---|---|---|
| 1. | Polypropylene glycol (1000 molecular weight) | 30.99 g |
| 2. | Polyethylene glycol (3000 molecular weight) | 60.49 g |
| 3. | Acetone (anhydrous) | 200.0 g |
| 4. | Methylenedi-p-phenyl diisocyanate (260 effective MW) | 75.12 g |
| 5. | Dibutyl tin dilaurate | 0.20 g |
| 6. | Dimethylol propionic acid | 33.43 g |
| 7. | Ethanol | 5.0 g |
| 8. | 1.0 N. Sodium Hydroxide | 100.0 mL |
| 9. | Deionized water as needed to bring reagent 8 to 300g | |

The emulsion was prepared following the procedure outlined in the synthesis of polyurethane A. The yield was 492.2g (36.7% solids). The neutralization equivalent of the emulsion was 0.273 meq/g. This corresponds to a neutralization equivalent of 0.744 meq/g on a 100% solids basis.
Polyurethane G. A polyurethane containing 5% by weight dimethylol propionic acid was prepared (total carboxylate functionality: 0.37 meq/g). The product was isolated as an emulsion in water.

| Reagents: | | |
|---|---|---|
| 1. | Polypropylene glycol (2000 molecular weight) | 97.82 g |
| 2. | Polyethylene glycol (8000 molecular weight) | 16.50 g |
| 3. | Dimethylol propionic acid | 7.50 g |
| 4. | 2-Butanone | 193.7 g |
| 5. | Methylenedi-p-phenyl diisocyanate (260 effective MW) | 28.28 g |
| 6. | Dibutyl tin dilaurate | 0.2 g |
| 7. | Ethanol | 5.0 g |
| 8. | 1.0 N. Sodium Hydroxide | 37.5 mL |
| 9. | Deionized water as needed to bring reagent 8 to 225g | |

The emulsion was prepared following the procedure outlined in the synthesis of polyurethane C. The yield was 277.5g (46.7% solids). The neutralization equivalent of the emulsion was 0.084 meq/g. This corresponds to a neutralization equivalent of 0.180 meq/g on a 100% solids basis.

### Hair Fixative Formulations

Each of the prepared polyurethane emulsions was formulated into an aerosol hair spray and compared to a control as follows. Each of the emulsions was initially diluted with water to a manageable viscosity and then neutralized with 2-amino-2-methyl-1-propanol (AMP) to bring the polyurethane into solution. The percentage neutralization was determined on the basis of the carboxylic acid monomer content of the polymer and was generally about 50-90% of the free acidity. The solution was then further diluted to 4 parts active polymer solids by the addition of dimethyl ether, or a blend of ethanol and ether. Formulations were made at 4 parts solids to attain equivalence with the percentage of polymer used in the control hair spray formulation. The polymer used in the control was a commercially available octyl-acrylamide/acrylatestt-btAylaminoethylmethacrylate copolymer. The formulations were tested for curl retention against the control according to the procedure described after the tables of results. The values for the curl retention test are given as percentages.

Polyurethane A exemplified the characteristics desired for hair fixative formulations. It formed clear films upon draw down, had a relatively misty spray pattern, and was soluble in low VOC systems. Therefore, it was formulated into 33% and 55% VOC systems (VOC percentage based on the total weight of the composition) as shown in Table 1 and tested for high humidity curl retention. The results are set out in Table 2 and show that polyurethane A performed better than the control in both the 33% and 55% VOC systems.

**Table 1**

| **Aerosol Formulations in Weight Percent** | | | | |
|---|---|---|---|---|
| VOC Formula | 33% VOC | 33% VOC | 55% VOC | 55% VOC |
| Polyurethane A | 10.65* | ---- | 10.65* | ---- |
| Control polymer | ---- | 4.00 | ---- | 4.00 |
| AMP | 00.24 | 00.79 | 00.24 | 00.79 |
| Ethanol | 00.00 | 00.00 | 22.00 | 22.00 |
| Water | 56.11 | 62.21 | 34.11 | 40.21 |
| DME | 33.00 | 33.00 | 33.00 | 33.00 |
| Total | 100.00 | 100.00 | 100.00 | 100.00 |

| | | | | |
|---|---|---|---|---|
| * corresponds to 4 parts active polymer | | | | |

**Table 2**

| **High Humidity Curl Retention at 90% RH, 21°C** | | | | | | | | | |
|---|---|---|---|---|---|---|---|---|---|
| 33%VOC | 15 min | 30 min | 60 min | 90 min | 2 hrs | 3 hrs | 4 hrs | 5 hrs | 24 hrs |
| A | 97.51+ | 95.00+ | 93.78+ | 91.27+ | 91.27+ | 91.27+ | 89.99+ | 89.99+ | 89.33+ |
| Control | 93.12 | 89.92 | 86.8 | 84.27 | 82.36 | 82.36 | 81.73 | 81.73 | 81.73 |

| 55%VOC | 15 min | 30 min | 60 min | 90 min | 2 hrs | 3 hrs | 4 hrs | 5 hrs | 24 hrs |
|---|---|---|---|---|---|---|---|---|---|
| A | 94.03+ | 91.42+ | 87.37+ | 84.75+ | 82.10+ | 78.78+ | 76.21 | 76.21 | 75.55 |
| Control | 89.67 | 84.08 | 80.67 | 77.80 | 75.84 | 73.04 | 71.69 | 71.69 | 70.99 |
| + indicates sample is superior to control at a 95% confidence level | | | | | | | | | |
| - indicates sample is inferior to control at a 95% confidence level | | | | | | | | | |
| otherwise there is no significant difference at a 95% confidence level | | | | | | | | | |

The polyurethane A 33% VOC system was also compared to the control for the characteristics of stiffness, resistance to combing, flake accumulation, gloss, static, length of time of initial tackiness, drying time, and shampoo removability. The polyurethane system was superior to the control in stiffness and resistance to combing at a 95% confidence level, and comparable to the control on the other characteristics.

The spray characteristics were also compared and polyurethane A showed reduced foaming in both the 55% and 33% VOC systems compared to the control. As was previously noted, many polymers developed for the current high VOC sprays increase in viscosity when used in aqueous ethanol systems. The viscosity is especially pronounced at higher solids. At 10% solids in water, with a #21 spindle, at 50 rpm and 25°C. polyurethane A had a viscosity of 13-16 mPa.s and the control had a viscosity of 109 mPa.s. The lower viscosity of the polyurethane is a contributing factor to the reduced foaming observed in the formulated 55% and 33% VOC systems.

Based on these results, the remaining polyurethanes B through G were formulated into 33% VOC aerosol systems and tested for curl retention. Polyurethane B contained 2% by weight of N-methyl diethanol amine and 14.6% by weight of dimethylol propionic acid (DMPA), making the polymer amphoteric. An aerosol formulation containing polyurethane B was neutralized to 60% and gave a clear solution that performed comparably to the control. Polyurethane C contained 24.6% DMPA and had low viscosity. When formulated into an aerosol, it performed equivalent to the control in curl retention and subjective properties (stiffness, flake accumulation, combing resistance, gloss, and static dissipation). Polyurethane D contained 7.5% by weight DMPA and showed a decrease in properties relative to the control. Polyurethane E contained 14.6% by weight of DMPA and was prepared from a cycloaliphatic diisocyanate. (Polyurethanes A-D were prepared from an aromatic diisocyanate.) Polyurethane E performed comparably to the control in curl retention. Polyurethane F was prepared without any polyethylene glycol and when formulated into an aerosol, performed comparably to the control. Polyurethane G was prepared with 5% by weight DMPA. After neutralization, it became an unstable opaque solution, and when formulated with dimethyl ether in an attempt to make an aerosol system, the system still contained excessive precipitate. Attempts to formulate polyurethane G into a 55% VOC system were also unsuccessful. The results are set out in the following tables.

**Table 3**

| **Aerosol Formulations in Weight Percent** | | | | | | | |
|---|---|---|---|---|---|---|---|
| Polyurethane | B | C | D | E | F | G | Control** |
| Polymer | 10.65* | 11.73* | 16.74* | 12.86* | 10.90* | 8.57* | 4.00* |
| AMP | 00.12 | 00.24 | 00.10 | 00.24 | 00.24 | 00.04 | 00.79 |
| Water | 56.23 | 55.03 | 50.16 | 53.90 | 55.86 | 58.39 | 62.21 |
| DME | 33.00 | 33.00 | 33.00 | 33.00 | 33.00 | 33.00 | 33.00 |
| Total | 100.00 | 100.00 | 100.00 | 100.00 | 100.00 | 100.00 | 100.00 |

| | | | | | | | |
|---|---|---|---|---|---|---|---|
| * corresponds to 4 parts active polymer | | | | | | | |
| ** commercially available Octylacrylamide/Acrylates/t-butylaminoethylemethacrylate Copolymer | | | | | | | |

**Table 4**

| **High Humidity Curl Retention at 90% RH, 21°C** | | | | | | | | | |
|---|---|---|---|---|---|---|---|---|---|
| Polymer | 15 min | 30 min | 60 min | 90 min | 2 hrs | 3 hrs | 4 hrs | 5 hrs | 6 hrs |
| Control | 97.35 | 96.03 | 94.53 | 93.66 | 91.42 | 90.39 | 90.35 | 89.85 | 90.43 |
| B | 97.87 | 96.80 | 95.57 | 94.77 | 94.82 | 94.65 | 95.08 | 94,18 | 94.00 |
| C | 94.90 | 94.76 | 95.77 | 94.35 | 93.91 | 92.71 | 92.18 | 91.98 | 90.98 |

**Table 5**

| **High Humidity Curl Retention at 90% RH, 21°C** | | | | | | | | | |
|---|---|---|---|---|---|---|---|---|---|
| Polymer | 15 min | 30 min | 60 min | 90 min | 2 hrs | 3 hrs | 4 hrs | 5 hrs | 24 hrs |
| Control | 89.86 | 87.69 | 84.07 | 84.35 | 84.48 | 82.10 | 82.29 | 80.31 | 78.55 |
| D | 90.55 | 87.49 | 81.98 | 80.60 | 78.19- | 76.68 | 74.39- | 71.92- | 45.72- |
| E | 94.19+ | 91.51 | 91.25+ | 87.12 | 86.12 | 86.76 | 85.89 | 85.59 | 80.51 |
| + indicates sample is superior to control at a 95% confidence level | | | | | | | | | |
| - indicates sample is inferior to control at a 95% confidence level | | | | | | | | | |
| otherwise there is no significant difference at a 95% confidence level. | | | | | | | | | |

**Table 6**

| **High Humidity Curl Retention at 90% RH, 21°C** | | | | | | | | | |
|---|---|---|---|---|---|---|---|---|---|
| Polymer | 15 min | 30 min | 60 min | 90 min | 2 hrs | 3 hrs | 4 hrs | 5 hrs | 6 hrs |
| Control | 93.12 | 89.92 | 86.80 | 84.27 | 82.36 | 82.36 | 81.73 | 81.73 | 81.73 |
| F | 93.07 | 90.61 | 86.84 | 85.61 | 84.37 | 83.74 | 83.10 | 83.10 | 82.46 |

Polyurethane E showed good properties in an aerosol formulation, and therefore was chosen as an exemplary polymer for formulation into an nonaerosol system. It was formulated into a completely aqueous system and compared to the control polymer for curl retention. The formulation and the test results are set out in Tables 7 and 8 and show that polyurethane E performed better than the control in the 0% VOC nonaerosol system.

**Table 7**

| **Non-aerosol Formulations in Weight Percent** | | |
|---|---|---|
| Polyurethane | E | Control** |
| Polymer | 12.86* | 4.00 |
| AMP | 00.26 | 00.98 |
| Water | 86.88 | 95.02 |
| | 100.00 | 100.00 |

| | | |
|---|---|---|
| * corresponds to 4 parts active polymer | | |
| **commercially available Octyfacrytamide/Actylates/t-Butylaminoethylmethacrylate Copolymer | | |

**Table 8**

| **Curl Retention** | | | | | | | | | |
|---|---|---|---|---|---|---|---|---|---|
| Polymer | 15 min | 30 min | 60 min | 90 min | 2 hr | 3 hr | 4 hr | 5 hr | 24 hr |
| E | 89.45+ | 83.41 + | 78.65+ | 78.00+ | 76.73+ | 76.73+ | 75.99+ | 75.99+ | 67.86+ |
| Control | 79.39 | 69.51 | 66.77 | 66.77 | 64.63 | 63.93 | 63.93 | 63.28 | 54.79 |
| + indicates sample is superior to control at a 95% confidence level | | | | | | | | | |
| - indicates sample is inferior to control at a 95% confidence level | | | | | | | | | |
| otherwise there is no significant difference at a 95% confidence level. | | | | | | | | | |

### Curl Retention Test Procedure

Each of the formulations prepared from Examples A to G was tested on nine swatches of strands of Remi Blue String European Brown hair for curl retention at 90% relative humidity, 22°C (72°F), and the results pooled and averaged. The testing procedure was as follows:

The hair was separated into swatches of approximately 2 grams in weight and bound at one end with cotton thread and epoxy glue. Each swatch was then washed in a 10% solution of shampoo, and rinsed in warm tap water. The hair was cut into 6 inch lengths from the secured end and dried at 49°C (120°F). It was wet again and combed, and the excess water squeezed out. The hair swatch was then rolled and secured onto a 1/2 inch diameter Teflon® mandrel, and dried at 49°C (120°F). When dried, it was removed from the mandrel and the resulting curl suspended by its bound end. For each swatch, the curl height was measured, and then the curl was sprayed uniformly with four sprays per side of nonaerosol formulation, or for two seconds per side with aerosol formulation. The curl was laid on a horizontal surface and allowed to air dry for one hour. The dried curl was then resuspended and set into a chamber at 22°C (72°F), 90% relative humidity, and the curl height measured immediately, and at 15, 30, 60 minute, and 2, 3, 4, 5, 6 and 24 hour intervals.

The percentage curl retention was calculated by the formula (L-L^{t})/(L-L^{o}) X 100, where L is the length of hair fully extended. L^{o} is the length of hair before spray and exposure, and L^{t} is the length of hair after spray and exposure.

### Description pour l'Etat contractant suivant: NL

This invention pertains to hair fixative compositions that are prepared from polyurethanes containing pendant free carboxyl groups that are neutralized with standard cosmetically acceptable bases.

Most hair fixative compositions contain a film-forming polymer, which acts as the fixative, and a delivery system, which is usually an alcohol or a mixture of alcohol and water. In the case of aerosol delivery, the delivery system will also contain a propellant, which is typically a volatile hydrocarbon. Due to environmental regulations controlling the emission of volatile organic compounds (VOCs) into the atmosphere, these alcohol and hydrocarbon delivery systems are becoming less acceptable, and it is foreseen that water will become a greater component in hair fixative compositions. However, many hair fixative polymers in current use exhibit a loss of performance properties in aqueous based systems; for example, the solution viscosity increases, and if delivered by aerosol, the composition foams at the valve actuator and on the hair. These factors have prompted the search for hair fixative polymers that are soluble in aqueous or in low VOC systems, which are systems that contain 80% or less VOCs, and that perform with all the desired characteristics of a good hair fixative polymer, namely, good holding power, high humidity curl retention, quick drying time, nonstickiness, and a clear, transparent, glossy film that is easily removable with water or with water and shampoo.

This invention is an aqueous based hair fixative composition that comprises an effective amount of a polyurethane to perform as a hair fixative in an all water or in an alcohol-water solvent system. Specifically, the hair fixative composition comprises (A) an effective amount of a fully reacted carboxylated linear polyurethane comprising the reaction product of (i) one or more 2,2-hydroxymethyl-substituted carboxylic acids present in an amount to give 0.35-2.25 milli equivalents of carboxyl functionality per gram of polyurethane, (ii) 10-90% by weight, based on the weight of the polyurethane, of one or more organic compounds having no more than two active hydrogen atoms each, and (iii) one or more organic diisocyanates present in a sufficient amount to react with the active hydrogens of the 2,2-hydroxymethyl-substituted carboxylic acids and the organic compounds, excepting the hydrogen on the carboxylate of the 2,2-hydroxymethyl-substituted carboxylic acid; (B) an effective amount of a cosmetically acceptable organic or inorganic base to neutralize a sufficient proportion of the available carboxyl groups on the polyurethane to make the polyurethane soluble in water or in a mixture of water and a polar organic solvent; and (C) a solvent comprising (i) water, and (ii) 0-85% by weight of a polar organic solvent, based on the weight of the solvent. In aerosol systems, the hair fixative composition will further comprise up to 60% by weight of a propellant based on the weight of the total hair fixative composition.

The polyurethanes suitable for use in hair fixative formulations according to this invention are fully reacted carboxylated linear polymers. These polyurethanes are used in an effective amount to achieve hair holding and humidity resistance properties. They are preferably present in amounts from 1-20% by weight of the hair fixative composition, and more preferably in amounts from 1-10% by weight. These polyurethanes are the reaction products of (i) one or more 2,2-hydroxymethyl-substituted carboxylic adds present in an amount to give 0.35-2.25 milliequivalents, preferably 0.5-1.85 milliequivalents, of carboxyl functionality per gram of polyurethane, (ii) 10-90% by weight, based on the weight of the polyurethane, of one or more organic compounds having no more than two active hydrogen atoms each, and (iii) one or more organic diisocyanates present in a sufficient amount to react with the active hydrogens of the 2,2-hydroxymethyl-substituted carboxylic acid and the organic compounds, excepting the hydrogen on the carboxylate of the 2,2-hydroxymethyl-substituted carboxylic acid. The incorporation of the 2,2-hydroxymethyl-substituted carboxylic acid introduces pendant carboxylic acid groups into the polymer chain, which after neutralization render the polyurethane soluble in water and in mixtures of water with other polar solvents. Using these polyurethanes as the active ingredient, hair fixative formulations can be made that have a high solids content with low viscosity. A high solids content supplies an effective amount of polymer to the hair in a minimum amount of solvent to obtain good holding power. Low viscosity permits effective atomization at the spray nozzle. Thus, a hair fixative product suitable for use in either aerosol or nonaerosol formulations can be achieved. The use of 2,2-hydroxymethyl-subsiituted carboxylic acids also imparts increased film hardness and rigidity to the polyurethane, properties that are desirable for hair fixatives.

The 2,2-hydroxymethyl-substituted carboxylic acids are represented by the formula 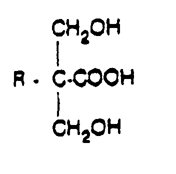 in which R represents hydrogen, or C₁ - C₂₀ alkyl, preferably C₁ - C₈ alkyl. Specific examples include 2,2-di(hydroxymethyl)acetic acid, 2,2-di(hydroxymethyl)propionic acid, 2,2-di-(hydroxymethyl)butyric acid, 2,2-di(hydroxymethyl)pentanoic acid, and the like. The preferred acid is 2,2-di-(hydroxymethyl)propionic acid. The 2,2-hydroxymethyl-substituted carboxylic acids are present in an amount to give 0.35-2.25, preferably 0.5-1.85, milliequivalents of carboxyl functionality per gram of polyurethane, and in general this is about 5-30% by weight of the polyurethane polymer.

The organic compounds that are reactive with isocyanate and that may be used for the preparation of the polyurethane polymers of this invention have no more than two active hydrogen atoms (as determined by the Zerewitinoff method). The active hydrogen atoms are usually attached to oxygen, nitrogen or sulfur atoms. These compounds will have a molecular weight of about 300 to 20,000, preferably about 500 to 8,000. Preferably, these compounds will be linear in order to prevent gelling during polymerization, but small amounts of non-linear compounds may be used provided their use does not cause gelling. The organic compounds will be present in an amount of 10-90% by weight of the polyurethane, preferably in an amount of 15-70% by weight.

The preferred organic compounds with two active hydrogen atoms are the linear difunctional polyethylene and polypropylene glycols, especially those that are available commercially and produced by the reaction of ethylene (or propylene) oxide with water, ethylene (or propylene) glycol, or diethylene (or dipropylene) glycol in the presence of sodium hydroxide as a catalyst. These polyglycols have molecular weights of about 600 to 20,000, preferably about 1,000 to 8.000. Polyglycols that are homogeneous in molecular weight, or a mixture of glycols that differ in molecular weight can be used. It is also possible to copolymerize small amounts of additional alkylene oxides into the polyglycol.

Other suitable organic compounds with two active hydrogen atoms are those having hydroxyl, carboxyl, amino or mercapto groups. Among these, the preferred are polyhydroxy compounds, such as, polyether diols, polyester diols, polyacetal diols, polyamide diols, polyester polyamide diols, poly(alkylene ether)diols, polythioether diols, and polycarbonate diols. Compounds that contain two or more different groups within these classes may also be used, for example, amino alcohols and amino alcohols containing two amino groups and one hydroxyl group. It is preferred to use difunctional compounds although small amounts of tri-(and greater) functional compounds may be used.

Suitable polyether diols are, for example, the condensation products of ethylene oxide, propylene oxide, butylene oxide, or tetrahydrofuran, and their copolymerization, graft or block polymerization products, such as, mixed ethylene oxide, propylene oxide condensates, and the graft polymerization products of the reaction of olefins under high pressure with the mentioned alkylene oxide condensates. Suitable polyethers are prepared by the condensation of the mentioned alkylene oxides with polyhydric alcohols, such as, ethylene glycol, 1,2-propylene glycol and 1,4-butanediol.

Suitable polyester diols, polyester amide diols, and polyamide diols are preferably saturated, and are obtained, for example, from the reaction of saturated or unsaturated polycarboxylic acids with saturated or unsaturated polyhydric alcohols, diamines, or polyamines. Suitable carboxylic acids for preparing these compounds include, for example, adipic acid, succinic acid, phthalic acid, terephthalic acid, and maleic acid. Suitable polyhydric alcohols for preparing the polyesters include, for example, ethylene glycol, 1,2-propylene glycol, 1,4-butanediol, neopentyl glycol, and hexanediol. Amino alcohols, for example, ethanclamine, are also useful. Suitable diamines for preparing the polyester amides and polyamides are, for example, ethylene diamine and hexamethylene diamine.

Suitable polyacetals can be prepared, for example, from 1,4-butanediol or hexanediol and formaldehyde. Suitable polythioethers can be prepared, for example, as the condensation products of thiodiglycol either alone or in combination with other glycols, such as, ethylene glycol, 1,2-propylene glycol or with other polyhydroxy compounds as disclosed above. Polyhydroxy compounds that already contain urea or urethane groups, and natural polyols, which may be further modified, for example, castor oil and carbohydrates, may also be used.

In preparing the polyurethane polymer, in addition to the organic compound having no more than two active hydrogen atoms, which in many cases is a high molecular weight compound, it may be desirable to chain extend the polymer using an organic compound with a lower molecular weight, preferably less than about 300 and more than 60. Typical chain extending agents include saturated or unsaturated glycols, such as, ethylene glycol, diethylene glycol, triethylene glycol and the like; amino alcohols, such as, ethanolamine, propanolamine, butanolamine, and the like; mono- and dialkoxylated aliphatic, cycloaliphatic, aromatic and heterocyclic primary amines, such as, N-mettryldiethanolamine, N-oleyl diethanolamine, N-cyclohexyl diisopropanolamine, N,N-dihydroxyethyl-p-toluidine, N,N-dihydroxy-propylnaphthylamine and the like; diamines, such as ethylene diamine, piperazine, N-N-bis-gamma-aminopropyl-N-methyl-amine and the like; carboxylic acids including aliphatic, cycloaliphatic, aromatic and heterocyclic dicarboxylic acids, such as, oxalic acid, malonic acid, succinic acid, glutaric acid, adipic acid, sebacic acid, terephthalic acid, 1,5-dicarboxylic naphthalic acid, maleic acid, fumaric acid, diglycolic acid, quinolinic acid, lutidinic acid and the like; amino carboxylic acids, such as, glycine, alpha and beta-alanine, 6-aminocaproic acid, 4-aminobutyric acid, p-aminobenzoic acid, 5-aminonaphthoic acid and the like. The preferred chain extending agents are aliphatic diols.

The organic polyisocyanates or mixtures of polyisocyanates that are reacted with the organic compound are aliphatic or aromatic polyisocyanates, or mixtures of those. The polyisocyanates are preferably diisocyanates in order to result in a linear polymer, although minor amounts of trifunctional isocyanates may be used in conjunction with the diisocyanates. The isocyanate will be present in a suffident amount to react with the active hydrogens of the 2,2-hydroxymethyl-substituted carboxylic acid and the organic compounds, excepting the hydrogen on the carboxylate of the 2,2-hydroxymethyl-substituted carboxylic acid. This amount will vary depending on the amounts of the carboxylic acid and organic compounds.

Exemplary diisocyanates include, but are not limited to, methylene-di-p-phenyl diisocyanate, methylene-bis(4-cyclohexylisocyanate), isophorone diisocyanate, toluene diisocyanate, 1,5-naphthalene diisocyanate, 4,4'-diphenylmethane diisocyanate, 2,2'-dimethyl-4,4'-diphenylmethane diisocyanate, 4,4'-dibenzyl-diisocyanate, 1,3-phenylene diisocyanate, 1,4-phenylene diisocyanate, mixtures of 2,4- and 2,6-toluene diisocyanate₁ 2,2'-dichloro-4,4'-diisocyanato diphenylmethane, 2,4-dibromo-1,5-diisocyanato naphthalene, butane-1,4-diisocyanate, hexane-1,6-diisocyanate, cyclohexane-1,4-diisocyanate.

If it is desired not to chain extend the polymer, the reaction of the diisocyanate with the organic compound having two active hydrogen atoms is quenched by the addition of a monofunctional active hydrogen-containing compound to consume any residual isocyanate functionality. Examples of these quenching compounds are well known in the art; for these systems, the preferred quenching compound is ethanol.

The urethane polymerization is carried out in the reaction medium with or without typical urethane reaction catalysts known in the art. Suitable catalysts include dibutyl tin dilaurate; the stannous salts of carboxylic acids having from 2 to 18 carbon atoms, such as, stannous laurate, stannous stearate, stannous acetate, stannous butyrate, stannous octoate and the like, and mixtures of those. Other suitable catalysts include dibutyl tin oxide, dibutyl tin sulfide, lead resinate, lead benzoate, lead salicylate, lead 2-ethyl hexoate, lead oleate, iron acetyl acetonate, cobalt benzoate, tetra (2-ethyl hexyl) titanate, tetra butyl titanate, and the like. Many other compounds accelerate the reaction of a hydroxyl or other groups with an isocyanate in preference to certain other reactions of the isocyanate group, and any of these compounds may be used. Those sldlled in the art will choose a specific catalyst to confer desired characteristics to individual urethane reactions. The preceding specific compounds are the preferred compounds and are given for the purpose of illustration and not limitation. In addition, any suitable tertiary amine may be used alone or with the metallic catalyst, for example, triethylene diamine, N-ethyl morpholine, N-methyl morpholine, or 4-dimethyl amino ethyl piperazine.

With respect to the proportion of reactants, the reactants should be selected so that the molecular ratio of isocyanate groups to active hydrogen atoms is as close to 1:1 as is practicable. It is appreciated that this exact ratio may not always be attained in practice; therefore, a ratio between about 0.7:1 and 1.3:1, and preferably between about 0.9:1 and 1.2:1, should be sought, and any excess diisocyanate, as discussed previously, can be quenched with the mono-functional active hydrogen containing compound.

The polymerization is carried out according to well known polyurethane polymerization techniques, which are well known to those skilled in the art. Exemplary polymerizations and reaction conditions are given in the examples.

To be used as hair fixatives, the carboxylated polyurethanes must be capable of being removed from the hair after use by a water rinse or shampoo. Removability is imparted by neutralizing the free carboxyl groups on the polyurethane. The amount of base used for neutralization is dependent on the hydrophobicity of the hair fixative polymer. The higher the carboxylic acid content of the polymer, the less the degree of neutralization required to impart water solubility. Conversely, the lower the carboxylic acid content, the greater the degree of neutralization required for water solubility. The preferred levels of neutralization range from 50-90%, depending on the acidity of the polymer. Suitable bases for neutralization of the polymer are the standard cosmetically acceptable bases known and used in the art. The preferred bases are sodium hydroxide, potassium hydroxide, 2-amino-2-methyl-1-propanol, histidine, tris(hydroxymethyl)aminomethane, and triethanolamine. The choice of the base and the degree of neutralization also affect the flexibility of the resultant hair fixative when sprayed on the hair, giving a soft or a hard hold. The choice of which base to utilize and the degree of neutralization required to achieve flexibility is within the expertise of one sldlled in the art. In general, however, the amount of base for neutralization will be within the range of 0.05-0.80% based on the total weight of the composition, although it will be recognized that individual formulations may require neutralization outside this range.

The neutralized polymers are soluble in water and, thus, the hair fixative compositions may be based solely in water, although more typically, the solvent system will be a blend of polar organic solvent and water. Typically, the organic solvent will be an alcohol or ketone. Particularly suitable solvents are low boiling alcohols that are compatible with other components in the hair fixative composition, for example, C₁-C₄ straight or branched chain alcohols. Exemplary polar solvents are ethanol, propanol, isopropanol, butanol, and acetone.

Hair fixative compositions that are intended to be delivered in an aerosol systems additionally will require a propellant. While any of the known propellants may be used in these compositions, preferred propellants include the hydrocarbons, particularly the lower boiling hydrocarbons such as C₃-C₆ straight and branched chain hydrocarbons, for example, propane, butane, isobutane and mixtures of those. Other preferred propellants include the ethers, such as dimethyl ether; hydrofluorocarbons, such as, 1,1 -difluoroethane; and the compressed gases, such as nitrogen, air and carbon dioxide. The amount of propellant used in the hair fixative compositions of this invention may vary from about 0 to 60% by weight of the hair spray composition and preferably from about 0 to 40% by weight, based on the weight of the total composition.

An important consideration in determining the amount of organic solvent, or organic solvent and propellant, to be used in the hair fixative composition is the total amount of volatile organic compound (VOC) content, and any upper limit of VOC content that may be mandated by environmental regulations. While these compositions may have a wide range of VOC content, from 0 to 85% by weight, it is preferred that there be less than about 80% and more preferably less than about 55% by weight VOC content, based on the weight of the composition. The balance of the hair fixative composition will be water and the neutralized polyurethane.

Optional conventional additives may also be incorporated into the hair fixing composition of this invention to provide certain modifying properties to the composition. Induded among these additives are plasticizers, such as glycerine, glycol and phthalate esters; silicones; emollients, lubricants and penetrants, such as lanolin compounds; fragrances and perfumes; UV absorbers; dyes and other colorants; thicfceners; anticorrosion agents; detacfdfying agents; combing aids; antistatic agents; preservatives; and foam stabilizers. These additives are present in small, effective amounts to accomplish their function, and generally will comprise from about 0.1 to 10% by weight each, and from about 0.1 to 20% by weight total, based on the weight of the composition.

The resulting hair fixing compositions exhibit all of the characteristics required of such a product in systems ranging from 0 to 85% VOC. The films found are clear, hard, glossy and provide humidity resistance while being readily removable.

### Examples

The following examples disclose the preparation of polyurethanes containing varying levels of dimethylol propionic acid and the results of humidity resistance testing on hair fixative formulations prepared from those polyurethanes.

### Syntheses of Polyurethanes

Polyurethane A. A polyurethane containing 16.7% by weight dimethylol propionic acid was prepared (total carboxylate functionality: 1.25 meq/g). The product was isolated as an emulsion in water.

| Reagents: | | |
|---|---|---|
| 1a. | Polypropylene glycol (1000 molecular weight) | 34.12 g |
| 1b. | Polypropylene glycol (3000 molecular weight) | 47.36 g |
| 2. | Polyethylene glycol (8000 molecular weight) | 10.00 g |
| 3. | Acetone (anhydrous) | 200.0 g |
| 4. | Methylenedi-p-phenyl diisocyanate (260 effective MW) | 75.12 g |
| 5. | Dibutyl tin dilaurate | 0.20 g |
| 6. | Dimethylol propionic acid | 33.40 g |
| 7. | Ethanol | 5.0 g |
| 8. | 1.0 N Sodium Hydroxide | 100.0 mL |
| 9. | Deionized water as needed to bring reagent 8 to 300g | |

A 1 L four-neck round bottom flask equipped with three stoppers and a vacuum adapter was charged with reagents 1 and 2. The reaction mixture was heated to 110°C under dynamic vacuum (<1 mm Hg) for 1 hour. The vacuum was released with nitrogen, and the flask was fit with a mechanical stirrer, thermometer, stopper, and a gas inlet-topped condenser. The reaction vessel was placed under a positive pressure of nitrogen and was maintained in this manner throughout the course of the urethane condensation reaction. The vessel was charged with reagent 3. The resulting mixture was brought to reflux and held there until a homogenous solution was obtained.

The reaction mixture was allowed to cool to less than 50°C. Reagents 4 and 5 were added, and the reaction was brought to reflux. The reaction was allowed to stir at reflux for 5 hours. Reagent 6 was added, and the reaction was stirred at reflux for an additional 11 hours. Reagent 7 was then added to the reaction mixture, and the reaction was allowed to stir at reflux for 1 hour.

Reagent 8 was charged into an addition funnel and then a sufficient quantity of reagent 9 was added to bring the total weight of reagent in the addition funnel to 300g. The contents of the funnel were added to the reaction mixture uniformly over a 3 hour period at 60°C. When the addition was complete, the flask was equipped for simple distillation, and distillation of solvent from the reaction mixture was started. When the pot temperature reached 90°C. steam distillation of the reaction mixture via the introduction of sub-surface steam was commenced. The steam distillation was continued until the pot temperature reached 100°C and was maintained at 100°C for 1 hour. A stable, fully aqueous emulsion was obtained at the conclusion of the steam distillation. The yield was 524.3g (37.6% solids). The neutralization equivalent of the emulsion was 0.286 meq/g. This corresponds to a neutralization equivalent of 0.761 meq/g on a 100% solids basis.
Polyurethane B. A polyurethane containing 14.6% by weight dimethylol propionic acid (total carboxylate functionality: 1.09 meq/g) and 2% by weight N-methyl diethanolamine was prepared. This polyurethane contains both cationic and anionic functionality. The product was isolated as an emulsion in water.

| Reagents: | | |
|---|---|---|
| 1. | Polyethylene glycol (2000 molecular weight) | 51.30 g |
| 2. | Polyethylene glycol (8000 molecular weight) | 16.57 g |
| 3. | Dimethylol propionic acid | 21.95 g |
| 4. | 2-Butanone | 190.4 g |
| 5. | Methylenedi-p-phenyl diisocyanate (260 effective MW) | 57.23 g |
| 6. | Dibutyl tin dilaurate | 0.20 g |
| 7. | N-methyl diethanolamine | 3.09 g |
| 8. | Ethanol | 5.0 g |
| 9. | 1.0 N Sodium hydroxide | 75.0 mL |
| 10. | Deionized water as needed to bring reagent 9 to 325g | |

A 1 L four-neck round bottom flask was equipped with a mechanical stirrer, thermometer, stopper, and a Dean & Stark-type receiver with draw-off valve fit with a gas inlet-topped condenser. The reaction vessel was placed under a positive pressure of nitrogen and was maintained in this manner throughout the course of the urethane condensation reaction. The vessel was charged with reagents 1, 2, 3, and 4. The resulting suspension was brought to reflux, and a total of 39.9g 2-butanone was distilled from the reaction vessel via the Dean & Stark receiver.

The reaction mixture was allowed to cool to less than 40°C. Reagents 5 and 6 added, and the reaction temperature was brought to 60°C. The reaction was allowed to stir at 60°C for 19 hours at which time the residual isocyanate was found to be 0.77% (theory: 0.81%). Reagent 7 was added at this time, and the reaction was stirred at 60°C for an additional 1.5 hours. The residual isocyanate was re-measured and found to be 0.11% (theory: 0.07%). Reagent 8 was then added to the reaction mixture, and the reaction was allowed to stir at 60°C for 1 hour.

Reagent 9 was charged into an addition funnel and then a sufficient quantity of reagent 10 was added to bring the total weight of reagent in the addition funnel to 325g. The contents of the funnel were added to the reaction mixture uniformly over a 3 hour period at 60°C. When the addition was complete, simple distillation at atmospheric pressure of solvent from the reaction mixture was started. When the pot temperature reached 90°C, steam distillation of the reaction mixture via the introduction of sub-surface steam was commenced. The steam distillation was continued until the pot temperature reached 100°C and was maintained at 100°C for 1 hour. A stable, fully aqueous emulsion was obtained at the conclusion of the steam distillation. The yield was 495.8g (28.6% solids). The neutralization equivalent of the emulsion was 0.167 meq/g. This corresponds to a neutralization equivalent of 0.584 meq/g on a 100% solids basis.
Polyurethane C. A polyurethane containing 24.6% by weight dimethylol propionic acid was prepared (total carboxylate functionality: 1.83 meq/g). The product was isolated as an emulsion in water.

| Reagents: | | |
|---|---|---|
| 1. | Polypropylene glycol (2000 molecular weight) | 21.47 g |
| 2. | Polyethylene glycol (8000 molecular weight) | 16.50 g |
| 3. | Dimethylol propionic acid | 36.31 g |
| 4. | 2-Butanone | 336.2 g |
| 5. | Methylenedi-p-phenyl diisocyanate (260 effective MW) | 75.83 g |
| 6. | Dibutyl tin dilaurate | 0.2g |
| 7. | Ethanol | 5.0 g |
| 8. | 1.0 N Sodium Hydroxide | 100.0 mL |
| 9. | Deionized water as needed to bring reagent 8 to 300g | |

A 1 L four-neck round bottom flask was equipped with a mechanical stirrer, thermometer, stopper, and a Dean & Stark-type receiver with draw-off valve fit with a gas inlet-topped condenser. The reaction vessel was placed under a positive pressure of nitrogen and was maintained in this manner throughout the course of the urethane condensation reaction. The vessel was charged with reagents 1, 2, 3, and 4. The resulting suspension was brought to reflux, and a total of 43g 2-butanone was distilled from the reaction vessel via the Dean & Stark receiver.

The reaction mixture was allowed to cool to less than 40°C. Reagents 5 and 6 were added, and the reaction temperature was brought to 60°C. The reaction was allowed to stir at 60°C for 20 hours at which time the residual isocyanate was found to be 0.05% (theory: 0.15%). Reagent 7 was added at this time, and the reaction was stirred at 60°C for an additional hour.

Reagent 8 was charged into an addition funnel and then a sufficient quantity of reagent 9 was added to bring the total weight of reagent in the addition funnel to 300g. The contents of the funnel were added to the reaction mixture uniformly over a 3 hour period at 60°C. When the addition was completed, simple distillation at atmospheric pressure of solvent from the reaction mixture was started. When the pot temperature reached 90°C, steam distillation of the reaction mixture via the introduction of sub-surface steam was commenced. The steam distillation was continued until the pot temperature reached 100°C and was maintained at 100°C for 45 minutes. A stable, fully aqueous emulsion was obtained at the conclusion of the steam distillation. The yield was 401.3g (34.1% solids). The neutralization equivalent of the emulsion was 0.286 meq/g. This corresponds to a neutralization equivalent of 0.839 meq/g on a 100% solids basis.
Polyurethane D. A polyurethane containing 7.5% by weight dimethylol propionic acid was prepared (total carboxylate functionality: 0.56 meq/g). The product was isolated as an emulsion in water.

| Reagents: | | |
|---|---|---|
| 1. | Polypropylene glycol (2000 molecular weight) | 87.55 g |
| 2. | Polyethylene glycol (8000 molecular weight) | 16.48 g |
| 3. | Dimethylol propionic acid | 11.26 g |
| 4. | 2-Butanone | 190.7 g |
| 5. | Methylenedi-p-phenyl diisocyanate (260 effective MW) | 34.68 g |
| 6. | Dibutyl tin dilaurate | 0.2 g |
| 7. | Ethanol | 5.0 g |
| 8. | 0.94 N Sodium Hydroxide | 35.8 mL |
| 9. | Deionized water as needed to bring reagent 8 to 275g | |

The emulsion was prepared following the procedure outlined for polyurethane Q. The yield was 611 g (23.9% solids). The neutralization equivalent of the emulsion was 0.080 meq/g. This corresponds to a neutralization equivalent of 0.33 meq/g on a 100% solids basis.
Polyurethane E. A polyurethane containing 14.6% by weight dimethyl propionic acid was prepared (total carboxylate functionality: 1.09 meq/g). A cycloaliphatic diisocyanate was used in the synthesis. The product was isolated as an emulsion in water.

| Reagents: | | |
|---|---|---|
| 1. | Polypropylene glycol (2000 molecular weight) | 63.48 g |
| 2. | Polyethylene glycol (8000 molecular weight) | 11.27 g |
| 3. | Dimethylol propionic acid | 22.03 g |
| 4. | 2-Butanone | 183.0 g |
| 5. | Methylene bis-(4-cyclohexyl isocyanate) (264 effective MW) | 53.90 g |
| 6. | Dibutyl tin dilaurate | 0.5 g |
| 7. | Ethanol | 10 g |
| 8. | 0.94 N Sodium Hydroxide | 34.6 mL |
| 9. | Deionized water as needed to bring reagent 8 to 275g | |

A 1 L four-neck round bottom flask was equipped with a mechanical stirrer, thermometer, stopper, and a Dean & Stark-type receiver with draw-off valve fit with a gas-inlet-topped condenser. The reaction vessel was placed under a positive pressure of nitrogen and was maintained in this manner throughout the course of the urethane condensation reaction. The vessel was charged with reagents 1, 2, 3, and 4. The resulting suspension was brought to reflux, and a total of 37.5g 2-butanone was distilled from the reaction vessel via the Dean & Stark receiver.

The reaction mixture was allowed to cool to less than 60°C. Reagents 5 and 6 were added, and the reaction temperature was brought to 70°-75°C. The reaction was allowed to stir at 70°-75°C for 30 hours at which time the residual isocyanate was found to be 0. 26% (theory: 0.20%). Reagent 7 was added at this time, and the reaction was stirred at 70°-75°C for an additional 10 hours.

Reagent 8 was charged into an addition funnel and then a sufficient quantity of reagent 9 was added to bring the total weight of reagent in the addition funnel to 275g. The contents of the funnel were added to the reaction mixture uniformly over a 3 hour period at 60°C. When the addition was complete, simple distillation at atmospheric pressure of solvent from the reaction mixture was started. When the pot temperature reached 90°C, steam distillation of the reaction mixture via the introduction of sub-surface steam was commenced. The steam distillation was continued until the pot temperature reached 100°C and was maintained at 100°C for 30 minutes. A stable, fully aqueous emulsion was obtained at the conclusion of the steam distillation. The yield was 458.1g (31.1% solids). The neutralization equivalent of the emulsion was 0.257 meq/g. This corresponds to a neutralization equivalent of 0.826 meq/g on a 100% solids basis.
Polyurethane F. A polyurethane containing 16.7% by weight dimethylol propionic acid was prepared (total carboxylate functionality: 1.25 meq/g). The product was isolated as an emulsion in water.

| Reagents: | | |
|---|---|---|
| 1. | Polypropylene glycol (1000 molecular weight) | 30.99 g |
| 2. | Polyethylene glycol (3000 molecular weight) | 60.49 g |
| 3. | Acetone (anhydrous) | 200.0 g |
| 4. | Methylenedi-p-phenyl diisocyanate (260 effective MW) | 75.12 g |
| 5. | Dibutyl tin dilaurate | 0.20 g |
| 6. | Dimethylol propionic acid | 33.43 g |
| 7. | Ethanol | 5.0 g |
| 8. | 1.0 N. Sodium Hydroxide | 100.0 mL |
| 9. | Deionized water as needed to bring reagent 8 to 300g | |

The emulsion was prepared following the procedure outlined in the synthesis of polyurethane A. The yield was 492.2g (36.7% solids). The neutralization equivalent of the emulsion was 0.273 meq/g. This corresponds to a neutralization equivalent of 0.744 meq/g on a 100% solids basis.
Polyurethane G. A polyurethane containing 5% by weight dimethylol propionic acid was prepared (total carboxylate functionality: 0.37 meq/g). The product was isolated as an emulsion in water.

| Reagents: | | |
|---|---|---|
| 1. | Polypropylene glycol (2000 molecular weight) | 97.82 g |
| 2. | Polyethylene glycol (8000 molecular weight) | 16.50 g |
| 3. | Dimethylol propionic acid | 7.50 g |
| 4. | 2-Butanone | 193.7 g |
| 5. | Methylenedi-p-phenyl diisocyanate (260 effective MW) | 28.28 g |
| 6. | Dibutyl tin dilaurate | 0.2 g |
| 7. | Ethanol | 5.0 g |
| 8. | 1.0 N. Sodium Hydroxide | 37.5 mL |
| 9. | Deionized water as needed to bring reagent 8 to 225g | |

The emulsion was prepared following the procedure outlined in the synthesis of polyurethane C. The yield was 277.5g (46.7% solids). The neutralization equivalent of the emulsion was 0.084 meq/g. This corresponds to a neutralization equivalent of 0.180 meq/g on a 100% solids basis.

### Hair Fixative Formulations

Each of the prepared polyurethane emulsions was formulated into an aerosol hair spray and compared to a control as follows. Each of the emulsions was initially diluted with water to a manageable viscosity and then neutralized with 2-amino-2-methyl-1-propanol (AMP) to bring the polyurethane into solution. The percentage neutralization was determined on the basis of the carboxylic acid monomer content of the polymer and was generally about 50-90% of the free acidity. The solution was then further diluted to 4 parts active polymer solids by the addition of dimethyl ether, or a blend of ethanol and ether. Formulations were made at 4 parts solids to attain equivalence with the percentage of polymer used in the control hair spray formulation. The polymer used in the control was a commerdally available octyl-acrylamide/acrylates/t-butylaminoethylmethacrylate copolymer. The formulations were tested for curl retention against the control according to the procedure described after the tables of results. The values for the curl retention test are given as percentages.

Polyurethane A exemplified the characteristics desired for hair fixative formulations. It formed clear films upon draw down, had a relatively misty spray pattern, and was soluble in low VOC systems. Therefore, it was formulated into 33% and 55% VOC systems (VOC percentage based on the total weight of the composition) as shown in Table 1 and tested for high humidity curl retention. The results are set out in Table 2 and show that polyurethane A performed better than the control in both the 33% and 55% VOC systems.

**Table 1**

| **Aerosol Formulations in Weight Percent** | | | | |
|---|---|---|---|---|
| VOC Formula | 33% VOC | 33% VOC | 55% VOC | 55% VOC |
| Polyurethane A | 10.65* | ---- | 10.65* | ---- |
| Control polymer | ---- | 4.00 | ---- | 4.00 |
| AMP | 00.24 | 00.79 | 00.24 | 00.79 |
| Ethanol | 00.00 | 00.00 | 22.00 | 22.00 |
| Water | 56.11 | 62.21 | 34.11 | 40.21 |
| DME | 33.00 | 33.00 | 33.00 | 33.00 |
| Total | 100.00 | 100.00 | 100.00 | 100.00 |

| | | | | |
|---|---|---|---|---|
| * corresponds to 4 parts active polymer | | | | |

**Table 2**

| **High Humidity Curl Retention at 90% RH, 21°C** | | | | | | | | | |
|---|---|---|---|---|---|---|---|---|---|
| 33%VOC | 15 min | 30 min | 60 min | 90 min | 2 hrs | 3 hrs | 4 hrs | 5 hrs | 24 hrs |
| A | 97.51+ | 95.00+ | 93.78+ | 91.27+ | 91.27+ | 91.27+ | 89.99+ | 89.99+ | 89.33+ |
| Control | 93.12 | 89.92 | 86.8 | 84.27 | 82.36 | 82.36 | 81.73 | 81.73 | 81.73 |

| 55%VOC | 15 min | 30 min | 60 min | 90 min | 2 hrs | 3 hrs | 4 hrs | 5 hrs | 24 hrs |
|---|---|---|---|---|---|---|---|---|---|
| A | 94.03+ | 91.42+ | 87.37+ | 84.75+ | 82.10+ | 78.78+ | 76.21 | 76.21 | 75.55 |
| Control | 89.67 | 84.08 | 80.67 | 77.80 | 75.84 | 73.04 | 71.69 | 71.69 | 70.99 |
| + indicates sample is superior to control at a 95% confidence level | | | | | | | | | |
| - indicates sample is inferior to control at a 95% confidence level | | | | | | | | | |
| otherwise there is no significant difference at a 95% confidence level | | | | | | | | | |

The polyurethane A 33% VOC system was also compared to the control for the characteristics of stiffness, resistance to combing, flake accumulation, gloss, static, length of time of initial tacldness, drying time, and shampoo removability. The polyurethane system was superior to the control in stiffness and resistance to combing at a 95% confidence level, and comparable to the control on the other characteristics.

The spray characteristics were also compared and polyurethane A showed reduced foaming in both the 55% and 33% VOC systems compared to the control. As was previously noted, many polymers developed for the current high VOC sprays increase in viscosity when used in aqueous ethanol systems. The viscosity is especially pronounced at higher solids. At 10% solids in water, with a #21 spindle, at 50 rpm and 25°C, polyurethane A had a viscosity of 13-16 mPa.s and the control had a viscosity of 109 mPa.s. The lower viscosity of the polyurethane is a contributing factor to the reduced foaming observed in the formulated 55% and 33% VOC systems.

Based on these results, the remaining polyurethanes B through G were formulated into 33% VOC aerosol systems and tested for curl retention. Polyurethane B contained 2% by weight of N-methyl diethanol amine and 14.6% by weight of dimethylol propionic acid (DMPA), making the polymer amphoteric. An aerosol formulation containing polyurethane B was neutralized to 60% and gave a clear solution that performed comparably to the control. Polyurethane C contained 24.6% DMPA and had low viscosity. When formulated into an aerosol, it performed equivalent to the control in curl retention and subjective properties (stiffness, flake accumulation, combing resistance, gloss, and static dissipation). Polyurethane D contained 7.5% by weight DMPA and showed a decrease in properties relative to the control. Polyurethane E contained 14.6% by weight of DMPA and was prepared from a cycloaliphatic diisocyanate. (Polyurethanes A-D were prepared from an aromatic diisocyanate.) Polyurethane E performed comparably to the control in curl retention. Polyurethane F was prepared without any polyethylene glycol and when formulated into an aerosol, performed comparably to the control. Polyurethane G was prepared with 5% by weight DMPA. After neutralization, it became an unstable opaque solution, and when formulated with dimethyl ether in an attempt to make an aerosol system, the system still contained excessive precipitate. Attempts to formulate polyurethane G into a 55% VOC system were also unsuccessful. The results are set out in the following tables.

**Table 3**

| **Aerosol Formulations in Weight Percent** | | | | | | | |
|---|---|---|---|---|---|---|---|
| Polyurethane | B | C | D | E | F | G | Control** |
| Polymer | 10.65* | 11.73* | 16.74* | 12.86* | 10.90* | 8.57* | 4.00* |
| AMP | 00.12 | 00.24 | 00.10 | 00.24 | 00.24 | 00.04 | 00.79 |
| Water | 56.23 | 55.03 | 50.16 | 53.90 | 55.86 | 58.39 | 62.21 |
| DME | 33.00 | 33.00 | 33.00 | 33.00 | 33.00 | 33.00 | 33.00 |
| Total | 100.00 | 100.00 | 100.00 | 100.00 | 100.00 | 100.00 | 100.00 |

| | | | | | | | |
|---|---|---|---|---|---|---|---|
| * corresponds to 4 parts active polymer | | | | | | | |
| ** commercially available Octylacrylamide/Acrylates/t-butylaminoethylemethacrylate Copolymer | | | | | | | |

**Table 4**

| **High Humidity Curl Retention at 90% RH, 21°C** | | | | | | | | | |
|---|---|---|---|---|---|---|---|---|---|
| Polymer | 15 min | 30 min | 60 min | 90 min | 2 hrs | 3 hrs | 4 hrs | 5 hrs | 6 hrs |
| Control | 97.35 | 96.03 | 94.53 | 93.66 | 91.42 | 90.39 | 90.35 | 89.85 | 90.43 |
| B | 97.87 | 96.80 | 95.57 | 94.77 | 94.82 | 94.65 | 95.08 | 94,18 | 94.00 |
| C | 94.90 | 94.76 | 95.77 | 94.35 | 93.91 | 92.71 | 92.18 | 91.98 | 90.98 |

**Table 5**

| **High Humidity Curt Retention at 90% RH, 21°C** | | | | | | | | | |
|---|---|---|---|---|---|---|---|---|---|
| Polymer | 15 min | 30 min | 60 min | 90 min | 2 hrs | 3 hrs | 4 hrs | 5 hrs | 24 hrs |
| Control | 89.86 | 87.69 | 84.07 | 84.35 | 84.48 | 82.10 | 82.29 | 80.31 | 78.55 |
| D | 90.55 | 87.49 | 81.98 | 80.60 | 78.19- | 76.68 | 74.39- | 71.92- | 45.72- |
| E | 94.19+ | 91.51 | 91.25+ | 87.12 | 86.12 | 86.76 | 85.89 | 85.59 | 80.51 |
| + indicates sample is superior to control at a 95% confidence level | | | | | | | | | |
| - indicates sample is inferior to control at a 95% confidence level | | | | | | | | | |
| otherwise there is no significant difference at a 95% confidence level. | | | | | | | | | |

**Table 6**

| **High Humidity Curl Retention at 90% RH, 21°C** | | | | | | | | | |
|---|---|---|---|---|---|---|---|---|---|
| Polymer | 15 min | 30 min | 60 min | 90 min | 2 hrs | 3 hrs | 4 hrs | 5 hrs | 6 hrs |
| Control | 93.12 | 89.92 | 86.80 | 84.27 | 82.36 | 82.36 | 81.73 | 81.73 | 81.73 |
| F | 93.07 | 90.61 | 86.84 | 85.61 | 84.37 | 83.74 | 83.10 | 83.10 | 82.46 |

Polyurethane E showed good properties in an aerosol formulation, and therefore was chosen as an exemplary polymer for formulation into an nonaerosol system. It was formulated into a completely aqueous system and compared to the control polymer for curl retention. The formulation and the test results are set out in Tables 7 and 8 and show that polyurethane E performed better than the control in the 0% VOC nonaerosol system.

**Table 7**

| **Non-aerosol Formulations in Weight Percent** | | |
|---|---|---|
| Polyurethane | E | Control** |
| Polymer | 12.86* | 4.00 |
| AMP | 00.26 | 00.98 |
| Water | 86.88 | 95.02 |
| | 100.00 | 100.00 |

| | | |
|---|---|---|
| * corresponds to 4 parts active polymer | | |
| **commercially available Octylacrylamide/Acrylates/t-Butylaminoethylmethacrylate Copolymer | | |

**Table 8**

| **Curl Retention** | | | | | | | | | |
|---|---|---|---|---|---|---|---|---|---|
| Polymer | 15 min | 30 min | 60 min | 90 min | 2 hr | 3 hr | 4 hr | 5 hr | 24 hr |
| E | 89.45+ | 83.41+ | 78.65+ | 78.00+ | 76.73+ | 76.73+ | 75.99+ | 75.99+ | 67.86+ |
| Control | 79.39 | 69.51 | 66.77 | 66.77 | 64.63 | 63.93 | 63.93 | 63.28 | 54.79 |
| + indicates sample is superior to control at a 95% confidence level | | | | | | | | | |
| - indicates sample is inferior to control at a 95% confidence level | | | | | | | | | |
| otherwise there is no significant difference at a 95% confidence level. | | | | | | | | | |

### Curl Retention Test Procedure

Each of the formulations prepared from Examples A to G was tested on nine swatches of strands of Remi Blue String European Brown hair for curl retention at 90% relative humidity, 22°C (72°F), and the results pooled and averaged. The testing procedure was as follows:

The hair was separated into swatches of approximately 2 grams in weight and bound at one end with cotton thread and epoxy glue. Each swatch was then washed in a 10% solution of shampoo, and rinsed in warm tap water. The hair was cut into 6 inch lengths from the secured end and dried at 49°C (120°F). It was wet again and combed, and the excess water squeezed out. The hair swatch was then rolled and secured onto a 1/2 inch diameter Teflon® mandrel, and dried at 49°C (120°F). When dried, it was removed from the mandrel and the resulting curl suspended by its bound end. For each swatch, the curl height was measured, and then the curl was sprayed uniformly with four sprays per side of nonaerosol formulation, or for two seconds per side with aerosol formulation. The curl was laid on a horizontal surface and allowed to air dry for one hour. The dried curl was then resuspended and set into a chamber at 22°C (72°F), 90% relative humidity, and the curl height measured immediately, and at 15, 30, 60 minute, and 2, 3. 4, 5, 6 and 24 hour intervals.

The percentage curl retention was calculated by the formula (L-L^{t})/(L-L^{o}) X 100, where L is the length of hair fully extended. L^{o} is the length of hair before spray and exposure, and L^{t} is the length of hair after spray and exposure.

## Claims (Claims for the following Contracting State(s): BE, DE, FR, GB, IT)

1. An aqueous based hair fixative composition that comprises
(A) an effective percent by weight, based on the total weight of the hair fixative composition, of a fully reacted carboxylated linear polyurethane comprising the reaction product of
(i) one or more 2,2-hydroxymethyl-substituted carboxylic acids, represented by the formula 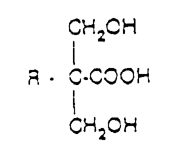 in which R represents hydrogen, or C₁ - C₂₀ alkyl, present in a sufficient amount by weight to give 0.35-2.25 milliequivalents of carboxyl functionality per gram of polyurethane,
(ii) 10-90% by weight, based on the weight of the polyurethane, of one or more organic compounds each having no more than two active hydrogen atoms, and
(iii) one or more organic diisocyanates to claim 1 selected from the group consisting of methylene-di-p-phenyl diisocyanate and methylene-bis-(4-cyclohexylisocyanate) selected from the group consisting of methylene-di-phenyl diisocyanate and methylene-bis-(4-cyclohexylisocyanate), present in a sufficient amount to react with the active hydrogens of the 2,2-hydroxymethyl-substituted carcoxyfic acid and the organic compounds, excepting the hydrogen on the carboxylate of the 2.2-hydroxymethyl-substituted carboxylic acid;
(B) an effective amount of one or more cosmetically acceptable organic or inorganic base to neutralize a sufficient proportion of the available carboxyl groups on the polyurethane to make the polyurethane soluble in water or in a mixture of water and polar organic solvent; and
(C) a solvent compnsing
(i) water, and
(ii) 0-85%, by weight of the solvent, of one or more polar organic solvents.

2. The hair fixative composition according to claim 1 in which the polyurethane is present in an amount from about 1-20% by weight of the hair fixative composition.

3. The hair fixative composition according to claim 1 in which the 2,2=hydroxymethyl-substituted carboxylic acid is present in an amount to give 0.5-1.85 milliequivalents per gram of polyurethane.

4. The hair fixative composition according to claim 1 in which the 2,2-hydroxymethyl-substituted carboxylic acid is 2,2-di-(hydroxymethyl)propionic acid.

5. The hair fixative composition according to c!aim 1 in which the organic compounds containing tvo active hydrogen atoms are present in an amount of 15-70% by weight of the polyurethane.

6. The hair fixative composition according to claim 1 in which the organic compounds containing two active hydrogen atoms are dicis.

7. The hair fixative composition according to claim 6 in which the diols are polyethylene glycol and polypropylene glycol.

8. The hair fixative composition according to claim 1 in which the organic compounds containing two active hydrogen atoms have a molecular weight of about 3C0 to 20,000.

9. The hair fixative composition according to claim 1 in which the organic compounds containing two active hydrogen atoms have a molecular weight of about 500 to 8,000.

10. The hair fixative composition according to claim 1 in which the neutralizing base is selected from the group consisting of sodium hydroxide, potassium hydroxide, 2-amino-2-methyl-1-propanol, histidine, tris(hydroxymethyl)aminomethane, and triethanolamine.

11. The hair fixative composition according to claim 1 in which the amount of base for neutralization is sufficient to neutralize 50-100% of the total acidity of the polymer.

12. The hair fixative composition according to claim 1 in which polar solvent is selected from the group consisting of ethanol, prcpanol, isopropanol, dimethyl ether, and acetone.

13. The hair fixative composition according to claim 1 in which the polar solvent is present in an amount up to 80% by weight of the total hair fixative composition.

14. The hair fixative composition according to claim 1 in which the polar solvent is present in an amount up to 55% by weight of the total hair fixative composition.

15. The hair fixative composition according to claim 1 in which the polar solvent is present in an amount up to 20% by weight of the total hair fixative composition.

16. The hair fixative composition according to claim 1 which further comprises up to 60% by weight of a propellant basad on the weight of the total hair fixative composition.

17. The hair fixative composition according to claim 16 in which the prcoellant is selected from the group consisting of dimethyl ether, C₃-C₅ straight and branched chain hydrocarbons, nydrofiucrscarccns, and compressed gases.

18. The hair fixative composition according to claim 16 in which the polyurethane is present in an amount from about 1-20% by weight of the hair fixative composition.

19. The hair fixative composition according to claim 16 in which the 2,2-hydroxymethyl-substituted carboxylic acid is present in an amount give 0.5-1.85 milliequivalents per gram of polyurethane.

20. The hair fixative composition according to claim 16 in which the 2,2-hydroxymethyl-substituted carboxylic acid is 2,2-di-(hydrcxymethyl)prcpionic acid.

21. The hair fixative composition according to claim 16 in which the organic compounds containing two active hydrogen atoms are present in an amount of 15-70% by weight of the polyurethane.

22. The hair fixative composition according to claim 16 in which the organic compounds containing two active hydrogen atoms are diols.

23. The hair fixative compcsition according to claim 16 in which the diets are selected from the group consisting of polyethylene glycol and polypropylene glycol.

24. The hair fixative composition according to claim 16 in which the organic compounds containing two active hydrogen atoms have a molecular weight of about 300 to 20,000.

25. The hair fixative composition according to claim 16 in which the organic compounds containing two active hydrogen atoms have a molecular weight of about 500 to 8,000.

26. The hair fixative composition according to claim 16 in which the organic diisocyanate is selected from the group consisting of methylenedi-p-phenyl diisocyanate and methylene-bis-(4-cyclohexylisocyanate).

27. The hair fixative composition according to claim 16 in which the neutralizing base is selected from the croup consisting of sodium hydroxide, potassium hydroxide. 2-amino-2-methyl-1-propanol, histidine, tris(hydroxymethyl)aminomethane, and triethanolamine.

28. The hair fixative composition according to claim 16 in which the amount of base for neutralization is sufficient to neutralize 50-100% of the total acidity of the polymer.

29. The hair fixative composition according to claim 16 in which polar solvent is selected from the group consisting of ethanol, propanol, isopropanol, dimethyl ether, and acetone.

30. The hair fixative compcsition according to claim 16 in which the polar solvent is present in an amount up to 80% by weight of the total hair fixative composition.

31. The hair fixative composition according to claim 16 in which the polar solvent is present in an amount up to 55% by weight of the total hair fixative composition.

32. The hair fixative composition according to claim 16 in which the polar solvent is present in an amount up to 20% by weight of the total hair fixative composition.

## Claims (Claims for the following Contracting State(s): NL)

1. An aqueous based hair fixative composition that comprises:
(A) an effective percent by weight, based on the total weight of the hair fixative composition, of a fully reacted carboxylated linear polyurethane comprising the reaction product of
(i) one or more 2,2-hydroxymethyl-substituted carboxylic acids, represented by the formula 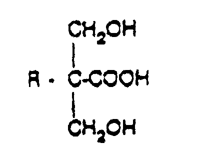 in which R represents hydrogen, or C₁ - C₂₀ alkyl, present in a sufficient amount by weight to give 0.35-2.25 milliequivalents of carboxyl functionality per gram of polyurethane,
(ii) 10-90% by weight, based on the weight of the polyurethane, of one or more organic compounds each having no more than two active hydrogen atoms, and
(iii) one or more organic diisocyanates present in a sufficient amount to react with the active hydrogens of the 2.2-hydroxymethyl-substituted carboxylic acid and the organic compounds, excepting the hydrogen on the carboxylate of the 2,2-hydroxymethyl-substituted carboxylic acid;
(B) an effective amount of one or more cosmetically acceptable organic or inorganic base to neutralize a sufficient proportion of the available carboxyl groups on the polyurethane to make the polyurethane soluble in water or in a mixture of water and polar organic solvent; and
(C) a solvent comprising
(i) water, and
(ii) 0-85%, by weight of the solvent, of one or more polar organic solvents.

2. The hair fixative composition according to claim 1 in which the polyurethane is present in an amount from about 1-20% by weight of the hair fixative composition.

3. The hair fixative composition according to claim 1 in which the 2,2=hydraxymethyl-substituted carboxylic acid is present in an amount to give 0.5-1.85 milliequivalents per gram of polyurethane.

4. The hair fixative composition according to claim 1 in which the 2,2-hydroxymethyl-substituted carboxylic acid is 2,2-di-{hydroxymethyl}propionic acid.

5. The hair fixative composition according to claim 1 in which the organic compounds containing two active hydrogen atoms are present in an amount of 15-70% by weight of the polyurethane.

6. The hair fixative composition according to claim 1 in which the organic compounds containing two active hydrogen atoms are diols.

7. The hair fixative composition according to claim 6 in which the diols are polyethylene glycol and polypropylene glycol.

8. The hair fixative composition according to claim 1 in which the organic compounds containing two active hydrogen atoms have a molecular weight of about 300 to 20,000.

9. The hair fixative composition according to claim 1 in which the organic compounds containing two active hydrogen atoms have a molecular weight of about 500 to 8,000.

10. The hair fixative composition according to claim 1 in which the organic diisocyanate is selected from the group consisting of methylenedi-p-phenyl diisocyante, methylene-bis-(4-cyclohexylisocyanate), isophorone diisocyanate, and toluene diisocyanate.

11. The hair fixative composition according to claim 1 in which the neutralizing base is selected from the group consisting of sodium hydroxide, potassium hydroxide, 2-amino-2-methyl-1-propanol, histidine, tris(hydroxymethyl)aminomethane, and triethanolamine.

12. The hair fixative composition according to claim 1 in which the amount of base for neutralization is sufficient to neutralize 50-100% of the total acidity of the polymer.

13. The hair fixative composition according to claim 1 in which polar solvent is selected from the group consisting of ethanol, propanol, isopropanol, dimethyl ether, and acetone.

14. The hair fixative composition according to claim 1 in which the polar solvent is present in an amount up to 80% by weight of the total hair fixative composition.

15. The hair fixative composition according to claim 1 in which the polar solvent is present in an amount up to 55% by weight of the total hair fixative composition.

16. The hair fixative composition according to claim 1 in which the polar solvent is present in an amount up to 20% by weight of the total hair fixative composition.

17. The hair fixative composition according to claim 1 which further comprises up to 60% by weight of a propellant based on the weight of the total hair fixative composition.

18. The hair fixative composition according to claim 17 in which the propellant is selected from the group consisting of dimethyl ether, C₃-C₆ straight and branched chain hydrocarbons, hydrofluorocarbons, and compressed gases.

19. The hair fixative composition according to claim 17 in which the polyurethane is present in an amount from about 1-20% by weight of the hair fixative composition.

20. The hair fixative composition according to daim 17 in which the 2,2-hydroxymethyl-substituted carboxylic acid is present in an amount t give 0.5-1.85 milliequivalents per gram of polyurethane.

21. The hair fixative composition according to claim 17 in which the 2,2-hydroxymethyl-substituted carboxylic acid is 2,2-di-(hydroxymethyl)propionic acid.

22. The hair fixative composition according to claim 17 in which the organic compounds containing two active hydrogen atoms are present in an amount of 15-70% by weight of the polyurethane.

23. The hair fixative composition according to claim 17 in which the organic compounds containing two active hydrogen atoms are diols.

24. The hair fixative composition according to claim 17 in which the diols are selected from the group consisting of polyethylene glycol and polypropylene glycol.

25. The hair fixative composition according to claim 17 in which the organic compounds containing two active hydrogen atoms have a molecular weight of about 300 to 20,000.

26. The hair fixative composition according to claim 17 in which the organic compounds containing two active hydrogen atoms have a molecular weight of about 500 to 8,000.

27. The hair fixative composition according to daim 17 in which the organic diisocyanate is selected from the group consisting of methylenedi-p-phenyl diisocyanate, methylene-bis-(4-cyclohexylisocyanate), isophorone diisocyanate, and toluene diisocyanate.

28. The hair fixative composition according to claim 17 in which the neutralizing base is selected from the group consisting of sodium hydroxide, potassium hydroxide, 2-amino-2-methyl-1-propanol, histidine, tris(hydroxymethyl)aminomethane, and triethanolamine.

29. The hair fixative composition according to claim 17 in which the amount of base for neutralization is sufficient to neutralize 50-100% of the total acidity of the polymer.

30. The hair fixative composition according to claim 17 in which polar solvent is selected from the group consisting of ethanol, propanol, isopropanol, dimethyl ether, and acetone.

31. The hair fixative composition according to claim 17 in which the polar solvent is present in an amount up to 80% by weight of the total hair fixative composition.

32. The hair fixative composition according to claim 17 in which the polar solvent is present in an amount up to 55% by weight of the total hair fixative composition.

33. The hair fixative composition according to claim 17 in which the polar solvent is present in an amount up to 20% by weight of the total hair fixative composition.

## Patentansprüche (Patentansprüche für folgende(n) Vertragsstaat(en): BE, DE, FR, GB, IT)

1. Wäßrige Haarfestigungszusammensetzung, die
(A) einen wirksamen Gewichtsprozentsatz, bezogen auf das Gesamtgewicht der Haarfestigungszusammensetzung, eines vollständig ausreagierten carboxylierten linearen Polyurethans, umfassend das Reaktionsprodukt aus
(i) einer oder mehreren 2,2-hydroxymethylsubstituierten Carbonsäure(n), dargestellt durch die Formel 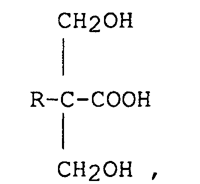 in der R Wasserstoff oder C₁-C₂₀-Alkyl bedeutet, vorhanden in einer Gewichtsmenge, die ausreicht, um 0,35-2,25 Milliäquivalent Carboxylfunktionalität pro Gramm Polyurethan zu ergeben,
(ii) 10-90 Gewichts-%, bezogen auf das Gewicht des Polyurethans, einer oder mehrerer organischen Verbindung(en), die jeweils nicht mehr als zwei aktive Wasserstoffatome aufweisen, und
(iii) einem oder mehreren organischen Diisocyanat(en), ausgewählt aus der Gruppe bestehend aus Methylendi-p-phenyldiisocyanat und Methylen-bis-(4-cyclohexylisocyanat), vorhanden in einer Menge, die ausreicht, um mit den aktiven Wasserstoffatomen der 2,2-hydroxymethylsubstituierten Carbonsäure und der organischen Verbindungen, mit Ausnahme des Wasserstoffs an dem Carboxylat der 2,2-hydroxymethylsubstituierten Carbonsäure, zu reagieren;
(B) eine die Neutralisation eines ausreichenden Anteils der verfügbaren Carboxylgruppen am Polyurethan bewirkende Menge einer oder mehrerer für kosmetische Zwecke zugelassenen organischen oder anorganischen Base(n), um das Polyurethan in Wasser oder in einem Gemisch aus Wasser und polarem organischem Lösungsmittel löslich zu machen, und
(C) ein Lösungsmittel, umfassend
(i) Wasser und
(ii) 0-85 Gewichts-%, bezogen auf das Lösungsmittel, eines oder mehrerer polaren organischen Lösungsmittel(s),
umfaßt.

2. Haarfestigungszusammensetzung nach Anspruch 1, in der das Polyurethan in einer Menge von etwa 1-20 Gewichts-%, bezogen auf die Haarfestigungszusammensetzung, vorliegt.

3. Haarfestigungszusammensetzung nach Anspruch 1, in der die 2,2-hydroxymethylsubstituierte Carbonsäure in einer Menge, die 0,5-1,85 Milliäquivalent pro Gramm Polyurethan ergibt, vorliegt.

4. Haarfestigungszusammensetzung nach Anspruch 1, in der die 2,2-hydroxymethylsubstituierte Carbonsäure 2,2-Di(hydroxymethyl)propionsäure ist.

5. Haarfestigungszusammensetzung nach Anspruch 1, in der die organischen Verbindungen, die zwei aktive Wasserstoffatome enthalten, in einer Menge von 15-70 Gewichts-%, bezogen auf das Polyurethan, vorliegen.

6. Haarfestigungszusammensetzung nach Anspruch 1, in der die organischen Verbindungen, die zwei aktive Wasserstoffatome enthalten, Diole sind.

7. Haarfestigungszusammensetzung nach Anspruch 6, in der die Diole Polyethylenglykol und Polypropylenglykol sind.

8. Haarfestigungszusammensetzung nach Anspruch 1, in der die organischen Verbindungen, die zwei aktive Wasserstoffatome enthalten, ein Molekulargewicht von etwa 300 bis 20.000 besitzen.

9. Haarfestigungszusammensetzung nach Anspruch 1, in der die organischen Verbindungen, die zwei aktive Wasserstoffatome enthalten, ein Molekulargewicht von etwa 500 bis 8.000 besitzen.

10. Haarfestigungszusammensetzung nach Anspruch 1, in der die neutralisierende Base aus der aus Natriumhydroxid, Kaliumhydroxid, 2-Amino-2-methyl-1-propanol, Histidin, Tris(hydroxymethyl)aminomethan und Triethanolamin bestehenden Gruppe ausgewählt ist.

11. Haarfestigungszusammensetzung nach Anspruch 1, in der die Menge der für die Neutralisation bestimmten Base ausreicht, um 50-100% der Gesamtazidität des Polymers zu neutralisieren.

12. Haarfestigungszusammensetzung nach Anspruch 1, in der das polare Lösungsmittel aus der aus Ethanol, Propanol, Isopropanol, Dimethylether und Aceton bestehenden Gruppe ausgewählt ist.

13. Haarfestigungszusammensetzung nach Anspruch 1, in der das polare Lösungsmittel in einer Menge von bis zu 80 Gewichts-% der gesamten Haarfestigungszusammensetzung vorliegt.

14. Haarfestigungszusammensetzung nach Anspruch 1, in der das polare Lösungsmittel in einer Menge von bis zu 55 Gewichts-% der gesamten Haarfestigungszusammensetzung vorliegt.

15. Haarfestigungszusammensetzung nach Anspruch 1, in der das polare Lösungsmittel in einer Menge von bis zu 20 Gewichts-% der gesamten Haarfestigungszusammensetzung vorliegt.

16. Haarfestigungszusammensetzung nach Anspruch 1, die ferner bis zu 60 Gewichts-%, bezogen auf das Gewicht der gesamten Haarfestigungszusammensetzung, eines Treibmittels umfaßt.

17. Haarfestigungszusammensetzung nach Anspruch 16, in der das Treibmittel aus der aus Dimethylether, gerade- und verzweigtkettigen C₃-C₆-Kohlenwasserstoffen, Fluorkohlenwasserstoffen und komprimierten Gasen bestehenden Gruppe ausgewählt ist.

18. Haarfestigungszusammensetzung nach Anspruch 16, in der das Polyurethan in einer Menge von etwa 1-20 Gewichts-% der Haarfestigungszusammensetzung vorliegt.

19. Haarfestigungszusammensetzung nach Anspruch 16, in der die 2,2-hydroxymethylsubstituierte Carbonsäure in einer Menge vorliegt, die 0,5-1,85 Milliäquivalent pro Gramm Polyurethan ergibt.

20. Haarfestigungszusammensetzung nach Anspruch 16, in der die 2,2-hydroxymethylsubstituierte Carbonsäure 2,2-Di(hydroxymethyl)propionsäure ist.

21. Haarfestigungszusammensetzung nach Anspruch 16, in der die organischen Verbindungen, die zwei aktive Wasserstoffatome enthalten, in einer Menge von 15-70 Gewichts-% des Polyurethans vorliegen.

22. Haarfestigungszusammensetzung nach Anspruch 16, in der die organischen Verbindungen, die zwei aktive Wasserstoffatome enthalten, Diole sind.

23. Haarfestigungszusammensetzung nach Anspruch 16, in der die Diole aus der aus Polyethylenglykol und Polypropylenglykol bestehenden Gruppe ausgewählt sind.

24. Haarfestigungszusammensetzung nach Anspruch 16, in der die organischen Verbindungen, die zwei aktive Wasserstoffatome enthalten, ein Molekulargewicht von etwa 300 bis 20.000 besitzen.

25. Haarfestigungszusammensetzung nach Anspruch 16, in der die organischen Verbindungen, die zwei aktive Wasserstoffatome enthalten, ein Molekulargewicht von etwa 500 bis 8.000 besitzen.

26. Haarfestigungszusammensetzung nach Anspruch 16, in der das organische Diisocyanat aus der aus Methylendi-p-phenyldiisocyanat und Methylen-bis(4-cyclohexyl) bestehenden Gruppe ausgewählt ist.

27. Haarfestigungszusammensetzung nach Anspruch 16, in der die neutralisierende Base aus der aus Natriumhydroxid, Kaliumhydroxid, 2-Amino-2-methyl-1-propanol, Histidin, Tris(hydroxymethyl)aminomethan und Triethanolamin bestehenden Gruppe ausgewählt ist.

28. Haarfestigungszusammensetzung nach Anspruch 16, in der die Menge an Base zur Neutralisation ausreicht, um 50-100 % der Gesamtazidität des Polymers zu neutralisieren.

29. Haarfestigungszusammensetzung nach Anspruch 16, in der das polare Lösungsmittel aus der aus Ethanol, Propanol, Isopropanol, Dimethylether und Aceton bestehenden Gruppe ausgewählt ist.

30. Haarfestigungszusammensetzung nach Anspruch 16, in der das polare Lösungsmittel in einer Menge von bis zu 80 Gewichts-% der gesamten Haarfestigungszusammensetzung vorliegt.

31. Haarfestigungszusammensetzung nach Anspruch 16, in der das polare Lösungsmittel in einer Menge von bis zu 55 Gewichts-% der gesamten Haarfestigungszusammensetzung vorliegt.

32. Haarfestigungszusammensetzung nach Anspruch 16, in der das polare Lösungsmittel in einer Menge von bis zu 20 Gewichts-% der gesamten Haarfestigungszusammensetzung vorliegt.

## Patentansprüche (Patentansprüche für folgende(n) Vertragsstaat(en): NL)

1. Wäßrige Haarfestigungszusammensetzung, die
(A) einen wirksamen Gewichtsprozentsatz, bezogen auf das Gesamtgewicht der Haarfestigungszusammensetzung, eines vollständig ausreagierten carboxylierten linearen Polyurethans, umfassend das Reaktionsprodukt aus
(i) einer oder mehreren 2,2-hydroxymethylsubstituierten Carbonsäure(n), dargestellt durch die Formel 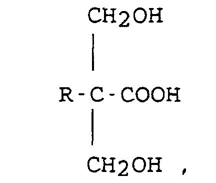 in der R Wasserstoff oder C₁-C₂₀-Alkyl bedeutet, vorhanden in einer Gewichtsmenge, die ausreicht, um 0,35-2,25 Milliäquivälent Carboxylfunktionalität pro Gramm Polyurethan zu ergeben,
(ii) 10-90 Gewichts-%, bezogen auf das Gewicht des Polyurethans, einer oder mehrerer organischen Verbindung(en), die jeweils nicht mehr als zwei aktive Wasserstoffatome aufweisen, und
(iii) einem oder mehreren organischen Diisocyanat(en), vorhanden in einer Menge, die ausreicht, um mit den aktiven Wasserstoffatomen der 2,2-hydroxymethylsubstituierten Carbonsäure und der organischen Verbindungen, mit Ausnahme des Wasserstoffs an dem Carboxylat der 2,2-hydroxymethylsubstituierten Carbonsäure, zu reagieren;
(B) eine die Neutralisation eines ausreichenden Anteils der verfügbaren Carboxylgruppen am Polyurethan bewirkende Menge einer oder mehrerer für kosmetische Zwecke zugelassenen organischen oder anorganischen Base(n), um das Polyurethan in Wasser oder in einem Gemisch aus Wasser und polarem organischem Lösungsmittel löslich zu machen, und
(C) ein Lösungsmittel, umfassend
(i) Wasser und
(ii) 0-85 Gewichts-%, bezogen auf das Lösungsmittel, eines oder mehrerer polaren organischen Lösungsmittel(s),
umfaßt.

2. Haarfestigungszusammensetzung nach Anspruch 1, in der das Polyurethan in einer Menge von etwa 1-20 Gewichts-%, bezogen auf die Haarfestigungszusammensetzung, vorliegt.

3. Haarfestigungszusammensetzung nach Anspruch 1, in der die 2,2-hydroxymethylsubstituierte Carbonsäure in einer Menge, die 0,5-1,85 Milliäquivalent pro Gramm Polyurethan ergibt, vorliegt.

4. Häarfestigungszusammensetzung nach Anspruch 1, in der die 2,2-hydroxymethylsubstituierte Carbonsäure 2,2-Di(hydroxymethyl)propionsäure ist.

5. Haarfestigungszusammensetzung nach Anspruch 1, in der die organischen Verbindungen, die zwei aktive Wasserstoffatome enthalten, in einer Menge von 15-70 Gewichts-%, bezogen auf das Polyurethan, vorliegen.

6. Haarfestigungszusammensetzung nach Anspruch 1, in der die organischen Verbindungen, die zwei aktive Wasserstoffatome enthalten, Diole sind.

7. Haarfestigungszusammensetzung nach Anspruch 6, in der die Diole Polyethylenglykol und Polypropylenglykol sind.

8. Haarfestigungszusammensetzung nach Anspruch 1, in der die organischen Verbindungen, die zwei aktive Wasserstoffatome enthalten, ein Molekulargewicht von etwa 300 bis 20.000 besitzen.

9. Haarfestigungszusammensetzung nach Anspruch 1, in der die organischen Verbindungen, die zwei aktive Wasserstoffatome enthalten, ein Molekulargewicht von etwa 500 bis 8.000 besitzen.

10. Haarfestigungszusammensetzung nach Anspruch 1, in der das organische Diisocyanat aus der aus Methylendi-p-phenyldiisocyanat, Methylen-bis(4-cyclohexylisocyanat), Isophorondiisocyanat und Toluoldiisocyanat bestehenden Gruppe ausgewählt ist.

11. Haarfestigungszusammensetzung nach Anspruch 1, in der die neutralisierende Base aus der aus Natriumhydroxid, Kaliumhydroxid, 2-Amino-2-methyl-1-propanol, Histidin, Tris(hydroxymethyl)aminomethan und Triethanolamin bestehenden Gruppe ausgewählt ist.

12. Haarfestigungszusammensetzung nach Anspruch 1, in der die Menge der für die Neutralisation bestimmten Base ausreicht, um 50-100% der Gesamtazidität des Polymers zu neutralisieren.

13. Haarfestigungszusammensetzung nach Anspruch 1, in der das polare Lösungsmittel aus der aus Ethanol, Propanol, Isopropanol, Dimethylether und Aceton bestehenden Gruppe ausgewählt ist.

14. Haarfestigungszusammensetzung nach Anspruch 1, in der das polare Lösungsmittel in einer Menge von bis zu 80 Gewichts-% der gesamten Haarfestigungszusammensetzung vorliegt.

15. Haarfestigungszusammensetzung nach Anspruch 1, in der das polare Lösungsmittel in einer Menge von bis zu 55 Gewichts-% der gesamten Haarfestigungszusammensetzung vorliegt.

16. Haarfestigungszusammensetzung nach Anspruch 1, in der das polare Lösungsmittel in einer Menge von bis zu 20 Gewichts-% der gesamten Haarfestigungszusammensetzung vorliegt.

17. Haarfestigungszusammensetzung nach Anspruch 1, die ferner bis zu 60 Gewichts-%, bezogen auf das Gewicht der gesamten Haarfestigungszusammensetzung, eines Treibmittels umfaßt.

18. Haarfestigungszusammensetzung nach Anspruch 17, in der das Treibmittel aus der aus Dimethylether, gerade- und verzweigtkettigen C₃―C₆-Kohlenwasserstoffen, Fluorkohlenwasserstoffen und komprimierten Gasen bestehenden Gruppe ausgewählt ist.

19. Haarfestigungszusammensetzung nach Anspruch 17, in der das Polyurethan in einer Menge von etwa 1-20 Gewichts-% der Haarfestigungszusammensetzung vorliegt.

20. Haarfestigungszusammensetzung nach Anspruch 17, in der die 2,2-hydroxymethylsubstituierte Carbonsäure in einer Menge vorliegt, die 0,5-1,85 Milliäquivalent pro Gramm Polyurethan ergibt.

21. Haarfestigungszusammensetzung nach Anspruch 17, in der die 2,2-hydroxymethylsubstituierte Carbonsäure 2,2-Di(hydroxymethyl)propionsäure ist.

22. Haarfestigungszusammensetzung nach Anspruch 17, in der die organischen Verbindungen, die zwei aktive Wasserstoffatome enthalten, in einer Menge von 15-70 Gewichts-% des Polyurethans vorliegen.

23. Haarfestigungszusammensetzung nach Anspruch 17, in der die organischen Verbindungen, die zwei aktive Wasserstoffatome enthalten, Diole sind.

24. Haarfestigungszusammensetzung nach Anspruch 17, in der die Diole aus der aus Polyethylenglykol und Polypropylenglykol bestehenden Gruppe ausgewählt sind.

25. Haarfestigungszusammensetzung nach Anspruch 17, in der die organischen Verbindungen, die zwei aktive Wasserstoffatome enthalten, ein Molekulargewicht von etwa 300 bis 20.000 besitzen.

26. Haarfestigungszusammensetzung nach Anspruch 17, in der die organischen Verbindungen, die zwei aktive Wasserstoffatome enthalten, ein Molekulargewicht von etwa 500 bis 8.000 besitzen.

27. Haarfestigungszusammensetzung nach Anspruch 17, in der das organische Diisocyanat aus der aus Methylendi-p-phenyldiisocyanat, Methylen-bis(4-cyclohexylisocyanat), Isophorondiisocyanat und Toluoldiisocyanat bestehenden Gruppe ausgewählt ist.

28. Haarfestigungszusammensetzung nach Anspruch 17, in der die neutralisierende Base aus der aus Natriumhydroxid, Kaliumhydroxid, 2-Amino-2-methyl-1-propanol, Histidin, Tris(hydroxymethyl)aminomethan und Triethanolamin bestehenden Gruppe ausgewählt ist.

29. Haarfestigungszusammensetzung nach Anspruch 17, in der die Menge an Base zur Neutralisation ausreicht, um 50-100 % der Gesamtazidität des Polymers zu neutralisieren.

30. Haarfestigungszusammensetzung nach Anspruch 17, in der das polare Lösungsmittel aus der aus Ethanol, Propanol, Isopropanol, Dimethylether und Aceton bestehenden Gruppe ausgewählt ist.

31. Haarfestigungszusammensetzung nach Anspruch 17, in der das polare Lösungsmittel in einer Menge von bis zu 80 Gewichts-% der gesamten Haarfestigungszusammensetzung vorliegt.

32. Haarfestigungszusammensetzung nach Anspruch 17, in der das polare Lösungsmittel in einer Menge von bis zu 55 Gewichts-% der gesamten Haarfestigungszusammensetzung vorliegt.

33. Haarfestigungszusammensetzung nach Anspruch 17, in der das polare Lösungsmittel in einer Menge von bis zu 20 Gewichts-% der gesamten Haarfestigungszusammensetzung vorliegt.

## Revendications (Revendications pour l'(les) Etat(s) contractant(s) suivant(s): BE, DE, FR, GB, IT)

1. Composition aqueuse de fixateur capillaire, qui comprend
(A) un pourcentage efficace, en poids, sur la base du poids total de la composition de fixateur capillaire, d'un polyuréthanne linéaire carboxylé, ayant réagi totalement, comprenant le produit de réaction
(i) d'un ou plusieurs acides carboxyliques à substituant 2,2-hydroxyméthyle, représentés par la formule 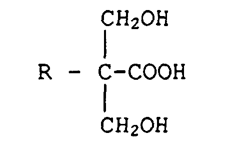 dans laquelle R représente l'hydrogène ou un groupe alkyle en C₁ à C₂₀,
présents en une quantité suffisante, en poids, pour fournir 0,35 à 2,25 milliéquivalents de fonctionnalité carboxyle par gramme de polyuréthanne,
(ii) de 10 à 90 % en poids, sur la base du poids du polyuréthanne, d'un ou plusieurs composés organiques n'ayant chacun pas plus de deux atomes d'hydrogène actifs, et
(iii) d'un ou plusieurs diisocyanates organiques choisis dans le groupe consistant en méthylènedi-p-phényldiisocyanate et méthylène-bis-(4-cyclohexylisocyanate, présents en une quantité suffisante pour la réaction avec les atomes d'hydrogène actifs de l'acide carboxylique à substituant 2,2-hydroxyméthyle et des composés organiques, à l'exception de l'atome d'hydrogène sur le carboxylate de l'acide carboxylique à substituant 2,2-hydroxyméthyle,
(B) une quantité efficace d'une ou plusieurs bases organiques ou inorganiques cosmétiquement acceptables pour neutraliser une proportion suffisante des groupes carboxyle disponibles sur le polyuréthanne afin de rendre le polyuréthanne soluble dans l'eau ou dans un mélange d'eau et d'un solvant organique polaire ;
(C) un solvant comprenant
(i) de l'eau, et
(ii) 0 à 85 %, en poids du solvant, d'un ou plusieurs solvants organiques polaires.

2. Composition de fixateur capillaire suivant la revendication 1, dans laquelle le polyuréthanne est présent en une quantité d'environ 1 à 20 % en poids de la composition de fixateur capillaire.

3. Composition de fixateur capillaire suivant la revendication 1, dans laquelle l'acide carboxylique à substituant 2,2-hydroxyméthyle est présent en une quantité fournissant 0,5 à 1,85 milliéquivalent par gramme de polyuréthanne.

4. Composition de fixateur capillaire suivant la revendication 1, dans laquelle l'acide carboxylique à substituant 2,2-hydroxyméthyle consiste en l'acide 2,2-dihydroxyméthylpropionique.

5. Composition de fixateur capillaire suivant la revendication 1, dans laquelle les composés organiques contenant 2 atomes d'hydrogène actifs sont présents en une quantité de 15 à 70 % en poids du polyuréthanne.

6. Composition de fixateur capillaire suivant la revendication 1, dans laquelle les composés organiques contenant 2 atomes d'hydrogène actifs sont des diols.

7. Composition de fixateur capillaire suivant la revendication 6, dans laquelle les diols consistent en polyéthylèneglycol et polypropylèneglycol.

8. Composition de fixateur capillaire suivant la revendication 1, dans laquelle les composés organiques contenant 2 atomes d'hydrogène actifs ont un poids moléculaire d'environ 300 à 20 000.

9. Composition de fixateur capillaire suivant la revendication 1, dans laquelle les composés organiques contenant 2 atomes d'hydrogène actifs ont un poids moléculaire d'environ 500 à 8000.

10. Composition de fixateur capillaire suivant la revendication 1, dans laquelle la base neutralisante est choisie dans le groupe consistant en hydroxyde de sodium, hydroxyde de potassium, 2-amino-2-méthyl-1-propanol, histidine, trishydroxyméthylaminométhane et triéthanolamine.

11. Composition de fixateur capillaire suivant la revendication 1, dans laquelle la quantité de base pour la neutralisation est suffisante pour neutraliser 50 à 100 % de l'acidité totale du polymère.

12. Composition de fixateur capillaire suivant la revendication 1, dans laquelle le solvant polaire est choisi dans le groupe consistant en éthanol, propanol, isopropanol, éther diméthylique et acétone.

13. Composition de fixateur capillaire suivant la revendication 1, dans laquelle le solvant polaire est présent en une quantité allant jusqu'à 80 % en poids de la composition de fixateur capillaire totale.

14. Composition de fixateur capillaire suivant la revendication 1, dans laquelle le solvant polaire est présent en une quantité allant jusqu'à 55 % en poids de la composition de fixateur capillaire totale.

15. Composition de fixateur capillaire suivant la revendication 1, dans laquelle le solvant polaire est présent en une quantité allant jusqu'à 20 % en poids de la composition de fixateur capillaire totale.

16. Composition de fixateur capillaire suivant la revendication 1, qui comprend en outre jusqu'à 60 % en poids d'un agent propulseur sur la base du poids de la composition de fixateur capillaire totale.

17. Composition de fixateur capillaire suivant la revendication 16, dans laquelle l'agent propulseur est choisi dans le groupe consistant en éther diméthylique, hydrocarbures droit et ramifié en C₃ à C₆, hydrofluorocarbones et gaz comprimés.

18. Composition de fixateur capillaire suivant la revendication 16, dans laquelle le polyuréthanne est présent en une quantité d'environ 1 à 20 % en poids de la composition de fixateur capillaire.

19. Composition de fixateur capillaire suivant la revendication 16, dans laquelle l'acide carboxylique à substituant 2,2-hydroxyméthyle est présent en une quantité fournissant 0,5 à 1,85 milliéquivalent par gramme de polyuréthanne.

20. Composition de fixateur capillaire suivant la revendication 16, dans laquelle l'acide carboxylique à substituant 2,2-hydroxyméthyle est l'acide 2,2-dihydroxyméthylpropionique.

21. Composition de fixateur capillaire suivant la revendication 16, dans laquelle les composés organiques contenant deux atomes d'hydrogène actifs sont présents en une quantité de 15 à 70 % en poids du polyuréthanne.

22. Composition de fixateur capillaire suivant la revendication 16, dans laquelle les composés organiques contenant deux atomes d'hydrogène actifs sont des diols.

23. Composition de fixateur capillaire suivant la revendication 16, dans laquelle les diols sont choisis dans le groupe consistant en polyéthylèneglycol et polypropylèneglycol.

24. Composition de fixateur capillaire suivant la revendication 16, dans laquelle les composés organiques contenant deux atomes d'hydrogène actifs ont un poids moléculaire d'environ 300 à 20 000.

25. Composition de fixateur capillaire suivant la revendication 16, dans laquelle les composés organiques contenant deux atomes d'hydrogène actifs ont un poids moléculaire d'environ 500 à 8000.

26. Composition de fixateur capillaire suivant la revendication 16, dans laquelle le diisocyanate organique est choisi dans le groupe consistant en méthylènedi-p-phényldiisocyanate et méthylène-bis-(4-cyclohexylisocyanate).

27. Composition de fixateur capillaire suivant la revendication 16, dans laquelle la base neutralisante est choisie dans le groupe consistant en hydroxyde de sodium, hydroxyde de potassium, 2-amino-2-méthyl-1-propanol, histidine, trishydroxyméthylaminométhane et triéthanolamine.

28. Composition de fixateur capillaire suivant la revendication 16, dans laquelle la quantité de base pour la neutralisation est suffisante pour neutraliser 50 à 100 % de l'acidité totale du polymère.

29. Composition de fixateur capillaire suivant la revendication 16, dans laquelle le solvant polaire est choisi dans le groupe consistant en éthanol, propanol, isopropanol, éther diméthylique et acétone.

30. Composition de fixateur capillaire suivant la revendication 16, dans laquelle le solvant polaire est présent en une quantité allant jusqu'à 80 % en poids de la composition de fixateur capillaire totale.

31. Composition de fixateur capillaire suivant la revendication 16, dans laquelle le solvant polaire est présent en une quantité allant jusqu'à 55 % en poids de la composition de fixateur capillaire totale.

32. Composition de fixateur capillaire suivant la revendication 16, dans laquelle le solvant polaire est présent en une quantité allant jusqu'à 20 % en poids de la composition de fixateur capillaire totale.

## Revendications (Revendications pour l'(les) Etat(s) contractant(s) suivant(s): NL)

1. Composition aqueuse de fixateur capillaire, qui comprend
(A) un pourcentage efficace, en poids, sur la base du poids total de la composition de fixateur capillaire, d'un polyuréthanne linéaire carboxylé, ayant réagi totalement, comprenant le produit de réaction
(i) d'un ou plusieurs acides carboxyliques à substituant 2,2-hydroxyméthyle, représentés par la formule 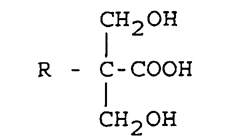 dans laquelle R représente l'hydrogène ou un groupe alkyle en C₁ à C₂₀,
présents en une quantité suffisante, en poids, pour fournir 0,35 à 2,25 milliéquivalents de fonctionnalité carboxyle par gramme de polyuréthanne,
(ii) de 10 à 90 % en poids, sur la base du poids du polyuréthanne, d'un ou plusieurs composés organiques n'ayant chacun pas plus de deux atomes d'hydrogène actifs, et
(iii) d'un ou plusieurs diisocyanates organiques présents en une quantité suffisante pour la réaction avec les atomes d'hydrogène actifs de l'acide carboxylique à substituant 2,2-hydroxyméthyle et des composés organiques, à l'exception de l'atome d'hydrogène sur le carboxylate de l'acide carboxylique à substituant 2,2-hydroxyméthyle,
(B) une quantité efficace d'une ou plusieurs bases organiques ou inorganiques cosmétiquement acceptables pour neutraliser une proportion suffisante des groupes carboxyle disponibles sur le polyuréthanne afin de rendre le polyuréthanne soluble dans l'eau ou dans un mélange d'eau et d'un solvant organique polaire ;
(C) un solvant comprenant
(i) de l'eau, et
(ii) 0 à 85 %, en poids du solvant, d'un ou plusieurs solvants organiques polaires.

2. Composition de fixateur capillaire suivant la revendication 1, dans laquelle le polyuréthanne est présent en une quantité d'environ 1 à 20 % en poids de la composition de fixateur capillaire.

3. Composition de fixateur capillaire suivant la revendication 1, dans laquelle l'acide carboxylique à substituant 2,2-hydroxyméthyle est présent en une quantité fournissant 0,5 à 1,85 milliéquivalent par gramme de polyuréthanne.

4. Composition de fixateur capillaire suivant la revendication 1, dans laquelle l'acide carboxylique à substituant 2,2-hydroxyméthyle consiste en l'acide 2,2-dihydroxyméthylpropionique.

5. Composition de fixateur capillaire suivant la revendication 1, dans laquelle les composés organiques contenant 2 atomes d'hydrogène actifs sont présents en une quantité de 15 à 70 % en poids du polyuréthanne.

6. Composition de fixateur capillaire suivant la revendication 1, dans laquelle les composés organiques contenant 2 atomes d'hydrogène actifs sont des diols.

7. Composition de fixateur capillaire suivant la revendication 6, dans laquelle les diols consistent en polyéthylène-glycol et polypropylèné-glycol.

8. Composition de fixateur capillaire suivant la revendication 1, dans laquelle les composés organiques contenant 2 atomes d'hydrogène actifs ont un poids moléculaire d'environ 300 à 20 000.

9. Composition de fixateur capillaire suivant la revendication 1, dans laquelle les composés organiques contenant 2 atomes d'hydrogène actifs ont un poids moléculaire d'environ 500 à 8000.

10. Composition de fixateur capillaire suivant la revendication 1, dans laquelle le diisocyanate organique est choisi dans le groupe consistant en méthylène-di-p-phényldiisocyanate, méthylène-bis-(4-cyclohexylisocyanate), isophorone-diisocyanate et toluène-diisocyanate.

11. Composition de fixateur capillaire suivant la revendication 1, dans laquelle la base neutralisante est choisie dans le groupe consistant en hydroxyde de sodium, hydroxyde de potassium, 2-amino-2-méthyl-1-propanol, histidine, trishydroxyméthylaminométhane et triéthanolamine.

12. Composition de fixateur capillaire suivant la revendication 1, dans laquelle la quantité de base pour la neutralisation est suffisante pour neutraliser 50 à 100 % de l'acidité totale du polymère.

13. Composition de fixateur capillaire suivant la revendication 1, dans laquelle le solvant polaire est choisi dans le groupe consistant en éthanol, propanol, isopropanol, éther diméthylique et acétone.

14. Composition de fixateur capillaire suivant la revendication 1, dans laquelle le solvant polaire est présent en une quantité allant jusqu'à 80 % en poids de la composition de fixateur capillaire totale.

15. Composition de fixateur capillaire suivant la revendication 1, dans laquelle le solvant polaire est présent en une quantité allant jusqu'à 55 % en poids de la composition de fixateur capillaire totale.

16. Composition de fixateur capillaire suivant la revendication 1, dans laquelle le solvant polaire est présent en une quantité allant jusqu'à 20 % en poids de la composition de fixateur capillaire totale.

17. Composition de fixateur capillaire suivant la revendication 1, qui comprend en outre jusqu'à 60 % en poids d'un agent propulseur sur la base du poids de la composition de fixateur capillaire totale.

18. Composition de fixateur capillaire suivant la revendication 17, dans laquelle l'agent propulseur est choisi dans le groupe consistant en éther diméthylique, hydrocarbures droit et ramifié en C₃ à C₆, hydrofluorocarbones et gaz comprimés.

19. Composition de fixateur capillaire suivant la revendication 17, dans laquelle le polyuréthanne est présent en une quantité d'environ 1 à 20 % en poids de la composition de fixateur capillaire.

20. Composition de fixateur capillaire suivant la revendication 17, dans laquelle l'acide carboxylique à substituant 2,2-hydroxyméthyle est présent en une quantité fournissant 0,5 à 1,85 milliéquivalent par gramme de polyuréthanne.

21. Composition de fixateur capillaire suivant la revendication 17, dans laquelle l'acide carboxylique à substituant 2,2-hydroxyméthyle est l'acide 2,2-dihydroxyméthylpropionique.

22. Composition de fixateur capillaire suivant la revendication 17, dans laquelle les composés organiques contenant deux atomes d'hydrogène actifs sont présents en une quantité de 15 à 70 % en poids du polyuréthanne.

23. Composition de fixateur capillaire suivant la revendication 17, dans laquelle les composés organiques contenant deux atomes.d'hydrogène actifs sont des diols.

24. Composition de fixateur capillaire suivant la revendication 17, dans laquelle les diols sont choisis dans le groupe consistant en polyéthylène-glycol et polypropylèneglycol.

25. Composition de fixateur capillaire suivant la revendication 17, dans laquelle les composés organiques contenant deux atomes d'hydrogène actifs ont un poids moléculaire d'environ 300 à 20 000.

26. Composition de fixateur capillaire suivant la revendication 17, dans laquelle les composés organiques contenant deux atomes d'hydrogène actifs ont un poids moléculaire d'environ 500 à 8000.

27. Composition de fixateur capillaire suivant la revendication 17, dans laquelle le diisocyanate organique est choisi dans le groupe consistant en méthylène-di-p-phényldiisocyanate, méthylène-bis-(4-cyclohexylisocyanate), isophorone-diisocyanate et toluène-diisocyanate.

28. Composition de fixateur capillaire suivant la revendication 17, dans laquelle la base neutralisante est choisie dans le groupe consistant en hydroxyde de sodium, hydroxyde de potassium, 2-amino-2-méthyl-1-propanol, histidine, trishydroxyméthylaminométhane et triéthanolamine.

29. Composition de fixateur capillaire suivant la revendication 17, dans laquelle la quantité de base pour la neutralisation est suffisante pour neutraliser 50 à 100 % de l'acidité totale du polymère.

30. Composition de fixateur capillaire suivant la revendication 17, dans laquelle le solvant polaire est choisi dans le groupe consistant en éthanol, propanol, isopropanol, éther diméthylique et acétone.

31. Composition de fixateur capillaire suivant la revendication 17, dans laquelle le solvant polaire est présent en une quantité allant jusqu'à 80 % en poids de la composition de fixateur capillaire totale.

32. Composition de fixateur capillaire suivant la revendication 17, dans laquelle le solvant polaire est présent en une quantité allant jusqu'à 55 % en poids de la composition de fixateur capillaire totale.

33. Composition de fixateur capillaire suivant la revendication 17, dans laquelle le solvant polaire est présent en une quantité allant jusqu'à 20 % en poids de la composition de fixateur capillaire totale.
